(19) Europäisches Patentamt
European Patent Office
Office européen des brevets

(11) Veröffentlichungsnummer : **0 030 702**
**B1**

(12) **EUROPÄISCHE PATENTSCHRIFT**

(45) Veröffentlichungstag der Patentschrift : **24.08.83**

(51) Int. Cl.³ : **C 07 C131/00**, A 01 N 39/02

(21) Anmeldenummer : **80107745.4**

(22) Anmeldetag : **09.12.80**

(54) **Propionsäureester, Herstellung dieser Verbindungen, Unkrautbekämpfungsmittel, die diese Verbindungen als Wirkstoffe enthalten sowie Verwendung solcher Verbindungen und Mittel zur Bekämpfung von Unkräutern.**

(30) Priorität : **12.12.79 CH 11003/79**
**28.10.80 CH 8018/80**

(43) Veröffentlichungstag der Anmeldung : **24.06.81 Patentblatt 81/25**

(45) Bekanntmachung des Hinweises auf die Patenterteilung : **24.08.83 Patentblatt 83/34**

(84) Benannte Vertragsstaaten : **AT BE CH DE FR IT LI NL SE**

(56) Entgegenhaltungen : **DE A 2 223 894**

(73) Patentinhaber : **F. HOFFMANN-LA ROCHE & CO. Aktiengesellschaft**
**CH-4002 Basel (CH)**

(72) Erfinder : **Fráter, Georg, Dr.**
**Am Pfisterhölzli 22**
**CH-8606 Greifensee (CH)**
Erfinder : **Wenger, Jean, Dr.**
**Brunnenwiesenstrasse 1**
**CH-8610 Uster (CH)**

(74) Vertreter : **Lederer, Franz, Dr. et al**
**Patentanwälte Dr. Franz Lederer Reiner F. Meyer**
**Lucile-Grahn-Strasse 22**
**D-8000 München 80 (DE)**

**EP 0 030 702 B1**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

# Propionsäureester, Herstellung dieser Verbindungen, Unkrautbekämpfungsmittel, die diese Verbindungen als Wirkstoffe enthalten sowie Verwendung solcher Verbindungen und Mittel zur Bekämpfung von Unkräutern

Die Erfindung betrifft Propionsäureester, nämlich Verbindungen der allgemeinen Formel

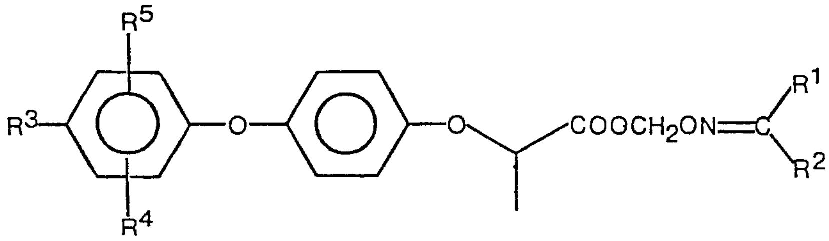

(I)

worin $R^1$ Wasserstoff, Alkyl mit 1-6 Kohlenstoffatomen oder Phenyl, $R^2$ Alkyl mit 1-6 Kohlenstoffatomen, Alkenyl mit 2-6 Kohlenstoffatomen, Alkinyl mit 2-6 Kohlenstoffatomen oder Phenyl, oder $R^1$ und $R^2$ zusammen mit dem Kohlenstoffatom, an das sie geknüpft sind, einen Cycloalkanring mit 4-10 Kohlenstoffatomen bilden, der gegebenenfalls mit Alkyl mit 1-3 Kohlenstoffatomen mono-, di- oder trisubstituiert sein kann, $R^3$ Halogen, Trifluormethyl oder Nitro und $R^4$ und $R^5$ Wasserstoff oder Halogen darstellen.

Die Erfindung betrifft ferner ein Verfahren zur Herstellung von Verbindungen der Formel I, (Vorauflauf- und Nachauflauf-)herbicide Mittel, Verfahren zur Herstellung solcher Mittel und zu deren Verwendung, sowie die Verwendung derartiger herbicider Mittel an sich.

Das Verfahren zur Herstellung von Verbindungen der Formel I ist dadurch gekennzeichnet, dass man

a) ein Salz einer Säure der Formel

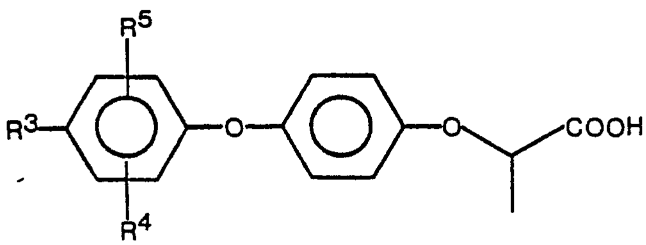

(II)

worin $R^3$, $R^4$ und $R^5$ die für Formel I angegebene Bedeutung besitzen, mit einer Verbindung der Formel

$$R^1R^2CNOCH_2X \qquad \text{(III)}$$

worin $R^1$ und $R^2$ die für Formel I angegebene Bedeutung besitzen und X eine Abgangsgruppe bedeutet, umsetzt, oder

b) eine Verbindung der Formel

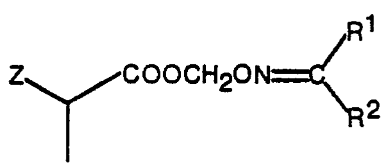

(IV)

worin Z eine Abgangsgruppe bedeutet und $R^1$ und $R^2$ die für Formel I angegebene Bedeutung besitzen, mit einer Verbindung der Formel

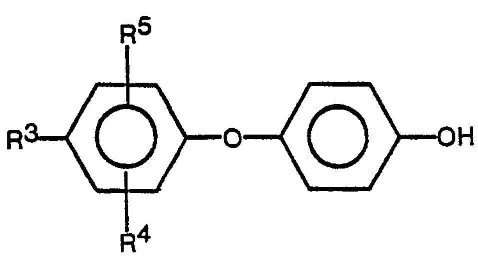

(V)

worin $R^3$, $R^4$ und $R^5$ die für Formel I angegebene Bedeutung besitzen, oder einem Alkalimetallsalz davon, notwendigenfalls in Gegenwart einer Base, umsetzt, oder

c) eine Verbindung der Formel

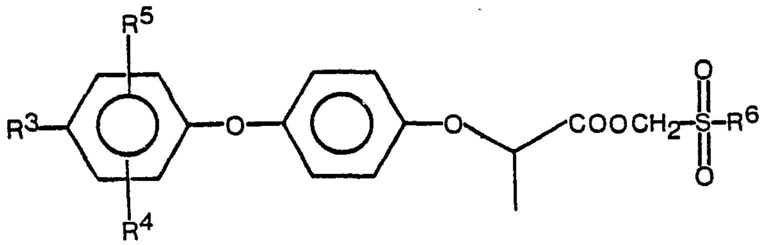

(VI)

worin $R^6$ eine nieder Alkyl-, Aryl- oder Heteroarylgruppe bedeutet und $R^3$, $R^4$ und $R^5$ die für Formel I angegebene Bedeutung besitzen, mit einer Verbindung der Formel

$$R^1R^2CNOH \quad (VII)$$

worin $R^1$ und $R^2$ die für Formel I angegebene Bedeutung besitzen, oder einem Alkalimetallsalz davon in Gegenwart einer Base umsetzt.

Auch die Deutsche Offenlegungsschrift 2.223.894 offenbart u. a. Alkylester von 2-Phenoxyphenoxypropionsäuren, die sich als herbizide Mittel eignen. Die erfindungsgemässen Verbindungen unterscheiden sich von den bekannten im wesentlichen dadurch, dass sie an der Alkylgruppe einen Ylidenaminooxy-Substituenten aufweisen.

Der Ausdruck « Alkyl » mit 1-6 bzw. 1-3 Kohlenstoffatomen umfasst — wenn nicht anderweitig angegeben — sowohl geradkettige als auch verzweigte Kohlenwasserstoffradikale mit 1-6 bzw. 1-3 Kohlenstoffatomen, wie Methyl, Aethyl, Propyl, Isopropyl, Butyl, Isobutyl, tert.-Butyl und dgl. Unter dem Ausdruck « nieder Alkyl » sind insbesondere Alkylgruppen mit 1-6 Kohlenstoffatomen zu verstehen.

Die Ausdrücke « Alkenyl » und « Alkinyl » mit 2-6 Kohlenstoffatomen umfassen ungesättigte geradkettige und verzweigte Kohlenwasserstoffradikale mit bis zu 6 Kohlenstoffatomen wie Allyl, Butenyl, Isobutenyl, Pentenyl, Isopentenyl und dgl. sowie Propargyl, Butinyl, Isobutinyl, Pentinyl und dgl. Besonders bevorzugte Alkinylgruppen sind 1-Propinyl, Butinyl und Isobutinyl.

Der Ausdruck « Halogen » umfasst Fluor, Chlor, Brom und Jod, vorzugsweise Chlor, Brom und Jod.

Der Cycloalkanring enthält vorzugsweise 4-7 Kohlenstoffatome, insbesondere 4-6 Kohlenstoffatome.

Unter « Arylgruppen » sind insbesondere Phenyl, gegebenenfalls mit 1-3 niederen Alkylgruppen substituiert, sowie Naphthyl zu verstehen. Die Alkylgruppen können gleich oder verschieden sein.

Bevorzugte « Heteroarylreste » sind der Pyridyl-, der Imidazolyl-, der Thiazolyl- und der Thiophenylrest.

Bevorzugte Verbindungen der Formel I sind solche, worin $R^1$ Methyl oder Aethyl bedeutet, solche, worin $R^2$ Methyl, Aethyl oder Phenyl bedeutet, solche, worin $R^3$ Chlor oder Trifluormethyl bedeutet, solche, worin $R^4$ Wasserstoff bedeutet und solche, worin $R^5$ Wasserstoff bedeutet. Auch die Verbindungen der Formel I, worin $R^1$ und $R^2$ zusammen mit dem Kohlenstoffatom, an das sie geknüpft sind, einen gegebenenfalls mit Alkyl substituierten Cycloalkanring bilden, sind bevorzugt.

Besonders bevorzugte Verbindungen der Formel I sind :

der 2-/p-[(α,α,α-Trifluor-p-tolyl)oxy]phenoxy/-propionsäure-/[(isopropylidenamino)oxy]methyl/ester und

der 2-[p-(p-Chlorphenoxy)phenoxy]-propionsäure-/[(isopropylidenamino)oxy]methyl/ester.

Weitere Beispiele für Verbindungen der Formel I sind :

der 2-[p-(2,4-Dichlorphenoxy)phenoxy]-propionsäure-/[(isopropylidenamino)-oxy]methyl/ester,

der 2-[p-(2,4-Dichlorphenoxy)phenoxy]-propionsäure-/[(α-methyl-benzylidenamino)-oxy]methyl/ester,

der 2-/p-[(α,α,α-Trifluor-p-tolyl)oxy]phenoxy/-propionsäure-/[(sec. butylidenamino)-oxy]methyl/ester,

der 2-/p-[(α,α,α-Trifluor-p-tolyl)oxy]phenoxy/-propionsäure-/[(1-pentyl-hexylidenamino)oxy]methyl/ester,

der 2-/p-[(α,α,α-Trifluor-p-tolyl)oxy]phenoxy/-propionsäure-/[(1.3-dimethyl-2-butylidenamino)oxy]methyl/ester,

der 2-/p-[(α,α,α-Trifluor-p-tolyl)oxy]phenoxy/-propionsäure-/[(α-methyl-benzylidenamino)oxy]methyl/ester,

der 2-/p-[(α,α,α-Trifluor-p-tolyl)oxy]phenoxy/-propionsäure-/[(cyclohexylidenamino)oxy]methyl/ester,

der 2-/p-[(α,α,α-Trifluor-p-tolyl)oxy]phenoxy/-propionsäure-/[(cyclopentylidenamino)oxy]methyl/ester,

der 2-/p-[(α,α,α-Trifluor-p-tolyl)oxy]phenoxy/-propionsäure-/[(cycloheptylidenamino)oxy]methyl/ester,

der 2-/p-[(α,α,α-Trifluor-p-tolyl)oxy]phenoxy/-propionsäure-/[(benzylidenamino)oxy]methyl/ester,

der 2-[p-(p-Bromphenoxy)phenoxy]-propionsäure-/[(isopropylidenamino)oxy]methyl/ester und

der 2-[p-(p-Nitrophenoxy)phenoxy]-propionsäure-/[(isopropylidenamino)oxy]methyl/ester,

sowie nieder-Alkyl-substituierte Derivate der oben genannten Cyclopentylidenamino-, Cyclohexylidenamino- und Cycloheptylidenamino-Verbindungen.

Besonders bevorzugt sind die D-Formen der Verbindungen der Formel I und somit die D-Formen der oben einzeln genannten Verbindungen.

In einer Ausführungsform zur Herstellung der Verbindungen der allgemeinen Formel I wird ein Salz einer Säure der Formel II mit einer Verbindung der Formel III umgesetzt.

Der Ausdruck « Salz einer Säure » bedeutet beispielsweise ein Alkalimetallsalz, wie beispielsweise Natrium-, Kalium- oder Lithiumsalz, oder ein Erdalkalimetallsalz, wie beispielsweise Magnesium-, Calcium- oder Bariumsalz, ein Salz einer organischen Base, wie beispielsweise ein Mono-, Di- oder Trialkylammoniumsalz oder ein Pyridiniumsalz, oder das Ammoniumsalz.

Der Ausdruck « Abgangsgruppe » für X steht beispielsweise für Chlor, Brom, Jod, Tosyloxy, Mesyloxy, Hydroxyl in freier oder veresterter Form, oder für eine der folgenden Gruppen :

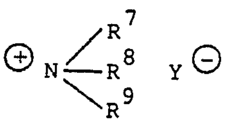

worin $R^7$, $R^8$ und $R^9$, welche Reste gleich oder verschieden sein können, Alkyl, insbesondere mit 1-6 Kohlenstoffatomen, bedeuten oder zwei davon auch zusammen eine $C_{4-7}$-Alkylengruppe bilden und $Y^{\ominus}$ ein Anion, z. B. ein Chlor-, Brom-, Jod-, Hydroxyl oder Sulfat-Anion, bedeutet ;

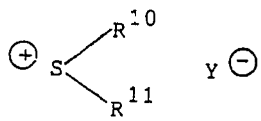

worin $R^{10}$ und $R^{11}$, welche Reste gleich oder verschieden sein können, Alkyl, insbesondere mit 1-6 Kohlenstoffatomen, bedeuten und $Y^{\ominus}$ die oben angegebene Bedeutung besitzt ;

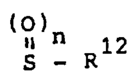

worin $R^{12}$ eine nieder Alkyl-, Aryl- oder Heteroarylgruppe und n 1 oder 2 bedeuten.

Die Veresterung der Säure der allgemeinen Formel II mit der Verbindung der allgemeinen Formel III wird vorzugsweise in einem geeigneten inerten Lösungsmittel, bei etwa − 10 bis 220 °C, bevorzugt bei Raumtemperatur oder erhöhter Temperatur, durchgeführt. Ein besonders bevorzugter Temperaturbereich liegt zwischen 20 °und 70 °C. Die Umsetzung wird beispielsweise in Gegenwart eines inerten Lösungsmittels durchgeführt, wie Benzol, Toluol, Petroläther, Dimethylformamid, Tetrahydrofuran, Acetonitril, N-Methyl-2-pyrrolidon, Tetramethylharnstoff, Dimethoxyäthan, Diglykoldimethyläther oder Hexamethylphosphorsäuretriamid.

In einer weiteren Ausführungsform zur Herstellung von Verbindungen der Formel I wird eine Verbindung der Formel IV mit einer Verbindung der Formel V oder einem Alkalimetallsalz einer Verbindung der Formel V in an sich bekannter Weise umgesetzt.

Der Ausdruck « Abgangsgruppe » für Z steht beispielsweise für eine der bereits für X angegebenen Abgangsgruppen, oder für eine durch Umsetzung mit Triphenylphosphin und Azodicarbonsäure oder einem Ester davon, insbesondere Azodicarbonsäure-diäthylester, aktivierte Hydroxygruppe (siehe z. B. Bull. Chem. Soc. Japan *46,* 2833 (1973) oder Angew. Chem. *88,* 111 (1976)).

Die Umsetzung gemäss Verfahrensvariante b) erfolgt zweckmässigerweise in einem inerten organischen Lösungsmittel wie Kohlenwasserstoffen, z. B. Benzol oder Toluol, Aethern, z. B. Diäthyläther, Tetrahydrofuran, Dimethoxyäthan oder Hexamethylphosphorsäuretriamid. Temperatur und Druck sind nicht kritisch, und man arbeitet bevorzugt bei einer Temperatur zwischen − 20 °C und der Rückflusstemperatur des Reaktionsgemisches, vorzugsweise zwischen − 10 und 30 °C.

In einer weiteren Ausführungsform zur Herstellung von Verbindungen der Formel I wird eine Verbindung der Formel VI mit einem Oxim der Formel VII in Gegenwart einer Base, beispielsweise einem Alkalimetallcarbonat, umgesetzt. Zu diesem Zweck kann die Verbindung der Formel VI in einem inerten organischen Lösungsmittel wie einem chlorierten Kohlenwasserstoff, beispielsweise Dichlormethan, Chloroform, Tetrachlorkohlenstoff oder Trichloräthan, einem Aether oder einer ätherartigen Verbindung, beispielsweise Tetrahydrofuran, Diäthyläther, Diisopropyläther, Dimethoxyäthan oder Dioxan, einem aromatischen Kohlenwasserstoff, beispielsweise Benzol, Toluol oder Xylol, Dimethylformamid, Dimethylsulfoxyd oder Hexamethylphosphorsäuretriamid gelöst werden, und danach wird das Oxim der Formel VII, beispielsweise in Form eines Alkalimetallsalzes, zugefügt. Die Umsetzung erfolgt in der Regel in einem Temperaturbereich zwischen 0 °C und der Siedetemperatur des Reaktionsgemisches, vorzugsweise zwischen 0° und 50 °C. Nach kurzer Zeit, z. B. wenigen Minuten, ist die Umsetzung normalerweise beendet. Das Reaktionsgemisch wird zweckmässigerweise auf Wasser gegossen und mit einem organischen Lösungsmittel, beispielsweise Aethylacetat, extrahiert. Der nach dem Eindampfen erhaltene Rückstand wird gegebenenfalls zur Reinigung umkristallisiert oder chromatographiert.

Die Verbindungen der allgemeinen Formel I schliessen auch optische Isomere ein, da sie ein

asymmetrisches Kohlenstoffatom in der α-Stellung zur Carbonylgruppe besitzen. Weitere asymmetrische Kohlenstoffatome können die Esterkomponenten enthalten. Erwünschtenfalls können die racemischen Verbindungen unter Verwendung bekannter Verfahren in rechtsdrehende und linksdrehende Verbindungen getrennt werden. Derartige Verfahren sind beispielsweise in Industrial and Engineering Chemistry *60* (8) 12-28 beschrieben. Die Isomeren und die racemischen Mischungen besitzen alle herbicide Aktivität, jedoch ist das Ausmass dieser Aktivität unterschiedlich. Am grössten ist die Aktivität beim D-Isomeren, danach folgt die racemische Mischung und dann das L-Isomere. Es wurde im Zusammenhang mit derartigen Untersuchungen gefunden, dass beispielsweise in bestimmten Versuchsanordnungen das D-Isomere des 2-/p[(α,α,α-Trifluor-p-tolyl)oxy]phenoxy/-propionsäure-/[(isopropylidenamino)-oxy]methyl]/esters eine grössere Aktivität als die racemische Mischung aufweist.

Die Isomeren können auch durch Synthese aus entsprechenden optisch aktiven Ausgangsmaterialien hergestellt werden. Die D-Isomeren der Verbindungen der Formel I sind, wie oben ausgeführt, besonders bevorzugt und somit auch die entsprechenden Ausgangsmaterialien.

Infolge der Stickstoff-Kohlenstoff-Doppelbindung in der

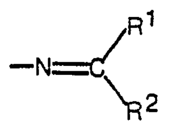

Gruppe erhält man jeweils zwei geometrische Isomere (wenn $R^1$ und $R^2$ unterschiedliche Bedeutung haben), die als syn- und anti-Form bezeichnet werden. Es gelingt in gewissen Fällen, derartige Isomere zu isolieren. Diese sind ebenfalls Gegenstand der Erfindung.

Die vorliegende Erfindung betrifft auch herbicide Zusammensetzungen, die als aktiven Bestandteil eine oder mehrere Verbindungen der Formel I, wie oben definiert, enthalten. Die herbicide Zusammensetzung enthält zweckmässigerweise zumindest eines der folgenden inerten Materialien : Trägerstoffe, Netzmittel, inerte Verdünnungsmittel und Lösungsmittel.

Die erfindungsgemässen (Vorauflauf- und Nachauflauf-) Herbicide eignen sich besonders zur Bekämpfung von Ungräsern, insbesondere von Ackerfuchsschwanz (Alopecurus myosuroides) und Hirsearten wie beispielsweise Hühnerhirse (Echinochloa crus-galli), grosse Borstenhirse (Setaria faberii) und haarartige Hirse (Panicum capillare) in Getreide — insbesondere Gerste-, Hafer- und Weizen- sowie Reis-, Baumwolle-, Soya-, Zuckerrüben- und Gemüsekulturen. Besonders bevorzugt eignen sich die erfindungsgemässen (Vorauflauf- und Nachauflauf-)Herbicide zur Bekämpfung von Ungräsern in Zukkerrübenkulturen. So besitzt beispielsweise der 2-/p-[(α,α,α-Trifluor-p-tolyl)oxyl]phenoxy/-propionsäure-/[(isopropylidenamino)oxy]methyl/ester bei einer Konzentration von 1,25 kg/ha genügend Wirksamkeit gegen Ungräser, ohne jedoch die Zuckerrübenkulturen zu schädigen. Im allgemeinen genügt eine Konzentration von 0,1-6 kg/ha, vorzugsweise 0,6-2,0 kg/ha, besonders bevorzugt 1-1,5 kg/ha, um den gewünschten herbiciden Effekt mit Verbindungen der Formel I zu erzielen.

Für den Fall, dass $R^3$ den Trifluormethylrest darstellt, können diese Verbindungen nur bedingt im Getreidebau Verwendung finden, da etwas Phototoxizität eintritt. Diese Verbindungen eignen sich jedoch besonders zur Bekämpfung von Ungräsern in Reis-, Baumwoll-, Soya-, Zuckerrüben- und Gemüsekulturen.

Die Verbindungen der Formel I sind im allgemeinen wasserunlöslich und können nach irgendeiner der für unlösliche Verbindungen üblichen Methoden konfektioniert werden.

Wenn gewünscht, können die Verbindungen der Formel I in einem mit Wasser nicht mischbaren Lösungsmittel, wie beispielsweise einem hochsiedenden Kohlenwasserstoff, gelöst werden, das zweckmässigerweise gelöste Emulgatoren enthält, so dass es bei Zugabe zu Wasser als selbstemulgierbares Oel wirkt.

Die Verbindungen der Formel I können auch mit einem Netzmittel mit oder ohne inertes Verdünnungsmittel zur Bildung eines netzbaren Pulvers vermischt werden, welches in Wasser löslich oder dispergierbar ist, oder sie können mit dem inerten Verdünnungsmittel zur Bildung eines festen oder pulverförmigen Produktes vermischt werden.

Inerte Verdünnungsmittel, mit welchen die Verbindungen der Formel I verarbeitet werden können, sind feste inerte Medien, einschliesslich pulverförmige oder feinverteilte Feststoffe, wie beispielsweise Tone, Sande, Talk, Glimmer, Düngemittel und dgl., wobei solche Produkte entweder staubförmig oder als Materialien mit grösserer Teilchengrösse vorliegen können.

Die Netzmittel können anionische Verbindungen, wie beispielsweise Seifen, Fettsulfatester, wie Dodecylnatriumsulfat, Octadecylnatriumsulfat und Cetylnatriumsulfat, fettaromatische Sulfonate, wie Alkylbenzolsulfonate oder Butylnaphthalinsulfonate, komplexere Fettsulfonate, wie die Amidkondensationsprodukte von Oelsäure und N-Methyltaurin oder das Natriumsulfonat von Dioctylsuccinat, sein.

Die Netzmittel können auch nichtionische Netzmittel, wie beispielsweise Kondensationsprodukte von Fettsäuren, Fettalkoholen oder fettsubstituierten Phenolen mit Aethylenoxyd, oder Fettsäureester und -äther von Zuckern oder mehrwertigen Alkoholen oder die Produkte, die aus letzteren durch Kondensation mit Aethylenoxyd erhalten werden, oder die Produkte, die als Blockcopolymere von Aethylenoxyd und

Propylenoxyd bekannt sind, sein. Die Netzmittel können auch kationische Mittel, wie beispielsweise Cetyltrimethylammoniumbromid und dgl., sein.

Die herbicide Zusammensetzung kann auch in Form eines Aerosols vorliegen, wobei zweckmässigerweise zusätzlich zum Treibgas, welches geeigneterweise ein polyhalogeniertes Alkan, wie Dichlordifluormethan, ist, ein Co-Solvens und ein Netzmittel verwendet werden.

Die herbiciden Zusammensetzungen gemäss der voliegenden Erfindung können zusätzlich zu den Verbindungen der Formel I Synergisten und andere Wirkstoffe, z. B. Insektizide, Akarizide, Bakterizide, anderweitige Herbicide, Fungizide, Pflanzenwachstumsregulatoren und Düngemittel, enthalten. Solche Kombinationsmittel eignen sich zur Verstärkung der Aktivität bzw. zur Verbreiterung des Wirkungsspektrums.

In ihren verschiedenen Anwendungsgebieten können die erfindungsgemässen Verbindungen in unterschiedlichen Mengenverhältnissen eingesetzt werden.

Die erfindungsgemässen Unkrautbekämpfungsmittel können in einer Form vorliegen, die sich für die Lagerung und den Transport eignen. Solche Formen können z. B. 2-90 Gewichtsprozent an einem oder mehreren Wirkstoffen der Formel I enthalten. Diese Formen können dann mit gleichen oder verschiedenen Trägermaterialien bis zu Konzentration verdünnt werden, die sich für den praktischen Gebrauch eignen. Im gebrauchsfertigen Mittel können dann Wirkstoffkonzentrationen von 0,05-80 Gewichtsprozent vorliegen. Die Wirkstoffkonzentration kann jedoch auch kleiner oder grösser sein. Je nach Verwendungszweck ist eine Wirkstoffkonzentration von 2-8 bzw. 50-80 Gewichtsprozent besonders bevorzugt.

Die Ausgangsmaterialien der Formeln II, III, IV, V und VII gehören bekannten Substanzklassen an.

Die Ausgangsmaterialien der Formel VI können durch Oxydation, beispielsweise mittels Wasserstoffsuperoxyd, von Verbindungen der Formel

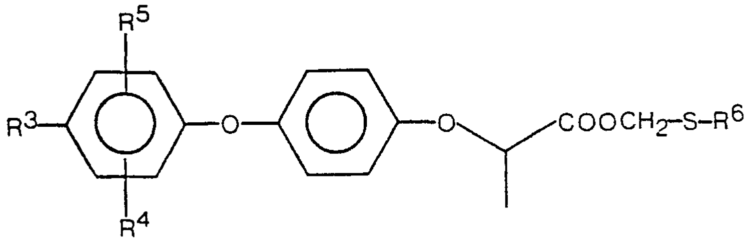

(VIII)

erhalten werden, worin $R^3$, $R^4$, $R^5$ und $R^6$ die oben angegebenen Bedeutungen besitzen.

Die Verbindungen der Formeln VI und VIII können in Analogie zu den Angaben in der DOS 2.617.804 hergestellt werden. So erhaltene Racemate können in üblicher Weise aufgespalten werden.

Die Verbindungen der Formeln VI und VIII, worin $R^6$ eine Heteroarylgruppe darstellt, sind neu, und zwar unabhängig davon, ob sie als Racemat oder in Form der optischen Antipoden, z. B. der D-antipoden, vorliegen. Bedeutet $R^6$ in den Verbindungen der Formeln VI und VIII eine niedere Alkylgruppe, z. B. die Methylgruppe, einen Arylrest, z. B. den Phenylrest oder einen Halogenphenylrest, so sind die entsprechenden D-Isomeren ebenfalls neu.

Diese neuen Verbindungen der Formeln VI und VIII können beispielsweise dadurch erhalten werden, dass man das Racemat oder die D-Form einer Verbindung der Formel II mit einer Verbindung der Formel

$$HO—CH_2—S—R^6 \qquad (IX)$$

umsetzt. Diese Umsetzung kann vorteilhaft in einem inerten organischen Lösungsmittel, beispielsweise in Chloroform oder Dimethylsulfoxyd, und in Gegenwart von Dicyclohexylcarbodiimid in an sich bekannter Weise durchgeführt werden. Eine so erhaltene Verbindung der Formel VIII kann dann, beispielsweise mit Wasserstoffsuperoxyd, zu einer Verbindung der Formel VI oxydiert werden.

Besonders bevorzugte Ausgangsmaterialien der Formeln VI bzw. VIII sind :

der D-2-[p-(p-(p-Bromphenoxy)phenoxy]-propionsäure-methylthiomethylester,
der D-2-[p-(p-(p-Fluorphenoxy)phenoxy]-propionsäure methylthiomethylester,
der D-2-[p-(p-Nitrophenoxy)phenoxy]-propionsäure methylthiomethylester und
der D-2-[p-(p-Trifluormethylphenoxy)phenoxy]-propionsäuremethylthiomethylester bzw. die entsprechenden Sulfonylverbindungen.

Die vorstehend angegebenen Ausgangsmaterialien der Formeln VI und VIII, insbesondere die D-Formen hiervon, sind zusätzlich auch als Herbicide wertvoll, da sie ein ähnliches Aktivitätsspektrum wie die Verbindungen der Formel I aufweisen. Sie können daher in der gleichen Weise, wie für die Verbindungen der Formel I beschrieben, für die Herstellung herbicider Mittel verwendet werden. Die D-Antipoden besitzen den Vorteil, dass sie im Vergleich zum jeweiligen Racemat beispielsweise eine geringere Phytotoxizität auf Baumwolle und Soyabohnen aufweisen.

Die folgenden Beispiele sollen die vorliegende Erfindung illustrieren. Alle Temperaturen sind in °C angegeben.

## I. Herstellung der Wirkstoffe der Formel I

### Beispiel 1

36 g 2-/p-[(α,α,α-Trifluor-p-tolyl)oxy]phenoxy/-propionsäure-[(methylsulfonyl)methyl]ester werden in 20 ml Tetrahydrofuran gelöst. Zu dieser Lösung wird ein Gemisch von 0,35 g Acetonoximnatriumsalz, gelöst in 20 ml Dimethylformamid, zugegeben und das Reaktionsgemisch 2 Minuten bei Raumtemperatur gerührt. Das Gemisch wird auf Wasser gegossen, mit Aethylacetat extrahiert und mit Wasser neutral gewaschen. Das Aethylacetat wird abgedampft und der Rückstand dann an 100 g Kieselgel mit Hexan/Aethylacetat 8 : 2 chromatographiert. Das Eluat wird eingedampft und der Rückstand aus Aether/Hexan umkristallisiert. Der erhaltene 2-/p-[(α,α,α-Trifluor-p-tolyl)oxy]phenoxy/-propionsäure-/[(isopropylidenamino)oxy]methyl/ester schmilzt bei 76-77°.

In analoger Weise erhält man beim Einsatz von

D-2-/p-[(α,α,α-Trifluor-p-tolyl)oxy]phenoxy/-propionsäure-[(methylsulfonyl)methyl]ester und Acetonoximnatriumsalz den D-2-/p-[(α,α,α-Trifluor-p-tolyl)oxy]phenoxy/-propionsäure-/[(isopropylidenamino) oxy]methyl/ester ; Fp. 80-85° ; $[\alpha]_d^{22}$ + 11,62° (c = 1,25 % in $CHCl_3$).

D-2-/p-[(α,α,α-Trifluor-p-tolyl)oxy]phenoxy/-propionsäure-[(methylsulfonyl)methyl]ester und Acetophenonoximnatriumsalz den D-2-/p-[(α,α,α-Trifluor-p-tolyl)oxy]phenoxy/-propionsäure-/[(α-methyl-benzylidenamino)oxy]methyl/ester ; Fp. 63-64° ; $[\alpha]_d^{22}$ – 13,64° (c = 0,75 % in $CHCl_3$).

D-2-[p-(p-Chlorphenoxy)phenoxy]-propionsäure-[(methylsulfonyl)methyl]ester und Acetonoximnatriumsalz den D-2-[p-(p-Chlorphenoxy)phenoxy]-propionsäure-/[(isopropylidenamino)oxy]methyl/ester ; $n_D^{20}$ 1,548 0 ; $[\alpha]_D^{22}$ + 13,58° (c = 1,93 % in $CHCl_3$).

2-[p-(o,p-Dichlorphenoxy)phenoxy]-propionsäure-[(methylsulfonyl)methyl]ester und Acetonoximnatriumsalz den 2-[p-(o,p-Dichlorphenoxy)phenoxy]-propionsäure-/[(isopropylidenamino)oxy]methyl/-ester ; $n_D^{20}$ 1,555 7.

2-[p-(o,p-Dichlorphenoxy)phenoxy]-propionsäure-[(methylsulfonyl)methyl]ester und Acetophenonoximnatriumsalz den 2-[p-(o,p-Dichlorphenoxy)phenoxy]-propionsäure-/[(α-methyl-benzylidenamino)-oxy]methyl/ester ; Fp. 66-67°.

D-2[p-(p-Bromphenoxy)phenoxy]-propionsäure-[(methylsulfonyl)methyl]ester und Acetonoximnatriumsalz den D-2-[p-(p-Bromphenoxy)phenoxy]-propionsäure-/[(isopropylidenamino)oxy]methyl/ester ; $n_D^{20}$ 1,559 8 ; $[\alpha]_D^{22}$ + 11,92° (c = 1,15 % in $CHCl_3$).

D-2-[p-(p-Jodphenoxy)phenoxy]-propionsäure-[(methylsulfonyl)methyl]ester und Acetonoximnatriumsalz den D-2-[p-(p-Jodphenoxy)phenoxy]-propionsäure-/[(isopropylidenamino)oxy]methyl/ester ; Fp. 40-43° ; $[\alpha]_D^{22}$ + 8,60° (c = 0,90 % in $CHCl_3$).

D-2-/p-[(α,α,α-Trifluor-p-tolyl)oxy]phenoxy/-propionsäure-[(methylsulfonyl)methyl]ester und Cyclopentanonoximnatriumsalz den D-2-/p-[(α,α,α-Trifluor-p-tolyl)oxy]-phenoxy/-propionsäure-/[cyclopentylidenamino)oxy]methyl/-ester ; Fp. 43-45° ; $[\alpha]_D^{22}$ + 7,52° (c = 2,28 % in $CHCl_3$).

D-2-/p-[(α,α,α-Trifluor-p-tolyl)oxy]phenoxy/propionsäure-[(methylsulfonyl)methyl/ester und Cyclohexanonoximnatriumsalz den D-2-/p-[(α,α,α-Trifluor-p-tolyl)oxy]-phenoxy/-propionsäure-/[(cyclohexylidenamino)oxy]methyl/-ester ; Fp. 71-73° ; $[\alpha]_D^{22}$ + 5,55° (c = 1,49 % in $CHCl_3$).

D-2-/p-[(α,α,α-Trifluor-p-tolyl)oxy]phenoxy/-propionsäure-[(methylsulfonyl)methyl]ester und Cycloheptanonoximnatriumsalz den D-2-/p-[(α,α,α-Trifluor-p-tolyl)oxy]-phenoxy/-propionsäure-/[(cycloheptylidenamino)oxy]methyl/-ester ; Fp. ca. 30° ; $[\alpha]_D^{22}$ + 6,00° (c = 1,96 % in $CHCl_3$).

D-2-/p-[(α,α,α-Trifluor-p-tolyl)oxy]phenoxy/-propionsäure-[(methylsulfonyl)methyl]ester und 3,3,5-Trimethylcyclohexanonoximnatriumsalz den D-2-/p-[(α,α,α-Trifluor-p-tolyl)oxy]phenoxy/-propionsäure-/[(3,3,5-trimethylcyclohexylidenamino)oxy]methyl/ester ; $n_D^{20}$ 1,506 2 ; $[\alpha]_D^{22}$ + 6,86° (c = 2,73 % in $CHCl_3$).

D-2-/p-[(α,α,α-Trifluor-p-tolyl)oxy]phenoxy/-propionsäure-[(methylsulfonyl)methyl ester und Benzaldoximnatriumsalz den D-2-/p-[(α,α,α-Trifluor-p-tolyl)oxy]phenoxy/-propionsäure-/[(benzylidenamino)oxy]-methyl/ester ; Fp. 65-66° ; $[\alpha]_D^{22}$ – 16,34° (c = 1,60 % in $CHCl_3$).

D-2-/p-[(α,α,α-Trifluor-p-tolyl)oxy]phenoxy/-propionsäure-[(methylsulfonyl)methyl]ester und Aethylmethylketoximnatriumsalz den D-2-/p-[(α,α,α-Trifluor-p-tolyl)oxy]phenoxy/-propionsäure-/[(sec. butylidenamino)oxy]methyl/ester ; Fp. 38-40° ; $[\alpha]_D^{22}$ + 10,34° (c = 2,36 % in $CHCl_3$).

D-2-/p-[(α,α,α-Trifluor-p-tolyl)oxy]phenoxy/-propionsäure-[(methylsulfonyl)methyl]ester und Dipentylketoximnatriumsalz den D-2-/p-[(α,α,α-Trifluor-p-tolyl)oxy]-phenoxy/-propionsäure-/[(1-pentyl-hexylidenamino)oxy]-methyl/ester ; $n_D^{20}$ 1,491 3 ; $[\alpha]_D^{22}$ + 11,71° (c = 3,27 % in $CHCl_3$).

D-2-/p-[(α,α,α-Trifluor-p-tolyl)oxy]phenoxy/-propionsäure-[(methylsulfonyl)methyl]ester und Methyl-(2-methyl-1-propenyl)-ketoximnatriumsalz den D-2-/p-[(α,α,α-Trifluor-p-tolyl)oxy]phenoxy/-propionsäure-/[(1,3-dimethyl-2-butenylidenamino)oxy]methyl/ester ; Fp. 52-54° ; $[\alpha]_D^{22}$ – 5,75° (c = 0,78 % in $CHCl_3$).

### Beispiel 2

Eine Lösung von 10 g (0,030 7 Mol) D-2-/p-[(α,α,α-Trifluor-p-tolyl)oxy]phenoxy/-propionsäure in 100 ml Dimethylformamid wird zu einer Suspension von 1,5 g 55 %iger (0,030 7 Mol) Natriumhybrid-Dispersion in 20 ml Dimethylformamid unter Rühren tropfenweise gegeben. Nach der Zugabe wird bei

Raumtemperatur weitergerührt, bis die Wasserstoffentwicklung beendet ist. Man rührt das Reaktionsgemisch noch 15 Minuten und gibt 15 g (0,048 Mol) N-[(Isopropylidenamino)-oxy]-N-methyl-piperidiniumjodid und 0,5 g 15-Crown-5 (die Polyäthylenäther-Kronenverbindung mit einem 5 Sauerstoffatome enthaltenden 15-Ring) zu. Nach 2-stündigem Rühren bei 110° wird das Reaktionsgemisch auf 500 ml Wasser gegossen und dreimal mit jeweils 300 ml Aethylacetat extrahiert. Die gesamte Aethylacetat-Lösung wird dann zweimal mit jeweils 200 ml Wasser gewaschen und abgedampft, und der Rückstand wird aus Diäthyläther/n-Hexan umkristallisiert. Man erhält 8,0 g D-2-/p-[(α,α,α-Trifluor-p-tolyl)oxy]phenoxy/-propionsäure-/[(isopropylidenamino)oxy]methyl/ester, der bei 85-86° schmilzt ; $[\alpha]_D^{20}$ + 11,3° (c = 1,2 % in $CHCl_3$).

Beispiel 3

1,75 g L(−)-Milchsäure-/[(isopropylidenamino)oxy]-methyl/ester, 2,65 g Triphenylphosphin und 2,54 g p-[(α,α,α-Trifluor-p-tolyl)oxy]-phenol werden bei 0° in 10 ml absolutem Tetrahydrofuran gelöst. Zu der Lösung werden unter Kühlung 1,75 g Azodicarbonsäure-diäthyläther getropft. Nach der Zugabe rührt man eine halbe Stunde nach, giesst dann das Reaktionsgemisch auf 100 ml Wasser, extrahiert es zweimal mit je 50 ml Wasser, trocknet es über wasserfreim Natriumsulfat und dampft es ein. Der Rückstand wird an der 20-fachen Menge Kieselgel mit n-Hexan/Aethylacetat (4 : 1) chromatographiert und das Eluat eingedampft. Man kristallisiert aus Diäthyläther/n-Hexan und erhält D-2-/p-[(α,α,α-Trifluor-p-tolyl)oxy]phenoxy/-propionsäure-/[(isopropylidenamino)-oxy]methyl/ester ; Fp. 85° ; $[\alpha]_D^{20}$ + 10,9° (c = 1,33 % in $CHCl_3$).

II. Herstellung der Ausgangsmaterialien

Beispiel 4

100 g 2-/p-[(α,α,α-Trifluor-p-tolyl) oxy]phenoxy/-propionsäure (0,03 Mol) und 252 g Natriumbicarbonat (3,0 Mol) werden in 2,5 l Dimethylsulfoxid suspendiert und danach langsam mit 336,5 ml tert. Butylbromid (3,0 Mol) versetzt. Man rührt über Nacht bei Raumtemperatur und nimmt das ganze Gemisch einschliesslich des ausgefallenen dicken Niederschlages in 5 l Essigester auf. Die Essigesterlösung wird zweimal mit 1 l Wasser gewaschen, über Natriumsulfat getrocknet und eingedampft. Der Rückstand wird an der 10-fachen Menge Kieselgel mit Hexan-Essigester im Verhältnis 7 : 1 chromatographiert und das Eluat eingedampft. Man erhält 2-/p-[(α,α,α-Trifluor-p-tolyl)oxy]Phenoxy/-propionsäure-[(methylthio) methyl]ester in Form eines Oels.

40 g 2-/p-[(α,α,α-Trifluor-p-tolyl)oxy]phenoxy/-propionsäure-[(methylthio)methyl]ester (0,096 Mol) werden in 120 ml Aceton/Wasser im Verhältnis 5 : 1 gelöst und langsam mit 192 ml Wasserstoffsuperoxyd (30 %ige Lösung) versetzt. Zum Gemisch werden 96 ml Ammoniummolybdatlösung (35,2 g Ammoniummolybdat in 96 ml Wasser) zugetropft, wobei mit einem Eisbad gekühlt wird. Danach wird das Gemisch 2 Stunden bei Zimmertemperatur gerührt. Es wird mehrfach mit Aether ausgeschüttelt, der Aetherextrakt mit Natriumdithionitlösung gewaschen und danach die Lösung nochmals achtmal mit jeweils 100 ml gesättigter Natriumchloridlösung und danach fünfmal mit jeweils 100 ml Wasser neutral gewaschen. Das Gemisch wird mit Aktivkohle behandelt, filtriert und eingedampft. Der Rückstand wird aus Methylenchlorid/n-Hexan umkristallisiert. Man erhält 2-/p-[(α,α,α-Trifluor-p-tolyl)oxy]-phenoxy/-propionsäure-[(Methylsulfonyl)methyl]ester, der bei 87-88° schmilzt.

Analog dem im ersten Absatz dieses Beispiels beschriebenen Verfahrens erhält man beim Einsatz von

D-2-/p-[(α,α,α-Trifluor-p-tolyl)oxy]phenoxy/-propionsäure den D-2-/p-[(α,α,α-Trifluor-p-tolyl)oxy]phenoxy/-propionsäure-[(methylthio)methyl]ester ; Fp. 47-48° ; $[\alpha\alpha]_D^{22}$ + 31,24° (c = 1,58 % in $CHCl_3$).

D-2-[p-(Chlorphenoxy)phenoxy]-propionsäure den D-2-[p-(p-Chlorphenoxy)phenoxy]-propionsäure-[(methylthio)-methyl]ester ; Fp. 75° ; $[\alpha]_D^{22}$ + 50,54° (c = 1,34 % in $CHCl_3$).

2-[p-(o,p-Dichlorphenoxy)phenoxy]-propionsäure den 2-[p-(o,p-Dichlorphenoxyl)phenoxy]-propionsäure-[(methylthio)methyl]ester ; Fp. 54°.

D-2-[p-(p-Bromphenoxy)phenoxy]-propionsäure den 2-[p-(o,p-Dichlorphenoxy)phenoxy]-propionsäure[(methylthio)-methyl]ester ; Fp. 83-87° ; $[\alpha]_D^{22}$ + 45,52° (c = 1,81 % in $CHCl_3$).

D-2-[p-(p-Jodphenoxy)phenoxy]-propionsäure den D-2-[p-(p-Jodphenoxy)phenoxy]-propionsäure-[(methylthio)methyl]-ester ; Fp. 71-74° ; $[\alpha]_D^{22}$ + 41,39° (c = 1,43 % in $CHCl_3$).

Analog dem im zweiten Absatz dieses Beispiels beschriebenen Verfahren erhält man beim Einsatz von

D-2-/p-[(α,α,α-Trifluor-p-tolyl)oxy]phenoxy/-propionsäure-[(methylthio)methyl]ester den D-2-/p-[(α,α,α-Trifluor-p-tolyl)oxy]phenoxy/-propionsäure-[(methylsulfonyl)-methyl]ester ; Fp. 79-82° ; $[\alpha]_D^{20}$ + 40,77° (c = 1,66 % in $CHCl_3$).

2-/p-[(α,α,α-Trifluor-p-tolyl)oxy]phenoxy/-propionsäure-[(phenylthio)methyl]ester den 2-/p[(α,α,α-Trifluor-p-tolyl)oxy]phenoxy/-propionsäure-[(phenylsulfonyl)methyl]-ester ; Fp. 89-92°.

D-2-[p-(p-Chlorphenoxy)phenoxy]-propionsäure-[(methylthio)methyl]ester den D-2-[p-(p-Chlorphe-

noxy)phenoxy]-propionsäure-[(methylsulfonyl)methyl]ester ; Fp. 81-83° ; $[\alpha]_D^{22}$ + 43,42° (c = 1,02 % in $CHCl_3$).

2-[p-(o,p-Dichlorphenoxy)phenoxy]-propionsäure-[(methylthio)methyl]ester den 2-[p-(o,p-Dichlorphenoxy)phenoxy]-propionsäure-[(methylsulfonyl)methyl]ester ; Fp. 95°.

D-2-[p-(p-Bromphenoxy)phenoxy]-propionsäure-[(methylthio)methyl]ester den D-2-[p-(Bromphenoxy)phenoxy]-propionsäure-[(methylsulfonyl)methyl]ester ; Fp. 84-87° ; $[\alpha]_D^{22}$ + 39,20° (c = 1,43 % in $CHCl_3$).

D-2-[p-(p-Jodphenoxy)phenoxy]-propionsäure-[(methylthio)methyl]ester den D-2-[p-(p-Jodphenoxy)phenoxy]-propionsäure-[(methylsulfonyl)methyl]ester ; Fp. 121-123° ; $[\alpha]_D^{22}$ + 37,91° (c = 2,66 % in $CHCl_3$).

## Beispiel 5

Zu einer Suspension von 10 g (0,11 Mol) L(+)-Milchsäure und 94,2 g (1,1 Mol) Natriumbicarbonat in 200 ml Dimethylsulfoxid werden 153 ml (1,1 Mol) tert. Butylbromid langsam zugetropft. Man rührt das Reaktionsgemisch 48 Stunden, giesst es danach auf 1 l Wasser und extrahiert es zweimal mit jeweils 100 ml Aethylacetat. Die organische Phase wird dreimal mit jeweils 200 ml Wasser nachgewaschen und anschliessend eingedampft. Das Rohprodukt wird an der 20-fachen Menge Kieselgel mit n-Hexan/Aethylacetat (4 : 1) chromatographiert und das Eluat eingedampft. Man erhält 5,7 g L(−)-Milchsäure-[(methylthio)methyl]ester, $[\alpha]_D^{20}$ − 39,27° (c = 1,3 % in $CHCl_3$).

Das obige Produkt kann auch erhalten werden, indem man 11,2 g L(+)-Natriumlactat, 0,5 g 15-Crown-5 und 9,25 g Chlordimethylsulfid sowie eine Spur Natriumjodid in 100 ml absolutem Acetonitril bei Rückflusstemperatur erhitzt. Nach 6 Stunden giesst man das Reaktionsgemisch auf Wasser und arbeitet es weiter wie oben auf. Man erhält 6,1 g L(−)-Milchsäure-[(methylthio)methyl]ester, $[\alpha]_D^{20}$ − 38,5° (c = 0,69 % in $CHCl_3$).

2,55 g (0,01 Mol) p-[(α,α,α-Trifluor-p-tolyl)oxy]-phenol, 1,5 g (0,01 Mol) L(−)-Milchsäure-[(methylthio)-methyl]ester und 2,63 g (0,01 Mol) Triphenylphosphin werden in 10 ml absolutem Tetrahydrofuran vorgelegt und das ganze auf 0° gekühlt. Unter Kühlung werden 0,011 Mol Azodicarbonsäure-diäthylester zugetropft, währenddessen die gelbe Farbe dieses Esters verschwindet. Das Reaktionsgemisch wird dann 30 Minuten bei Raumtemperatur gerührt, auf 100 ml Wasser gegossen, zweimal mit jeweils 50 ml Aethylacetat extrahiert und die organische Phase mit Wasser neutral gewaschen. Danach wird die Aethylacetat-Lösung eingedampft, der Rückstand an der 20-fachen Menge Kieselgel mit n-Hexan/Aethylacetat (9 : 1) chromatographiert und das Eluat eingedampft. Nach Kristallisieren aus Diäthyläther/n-Hexan erhält man 2,1 g D-2-/p-[(α,α,α-Trifluor-p-tolyl)oxy]phenoxy/-propionsäure-[(methylthio)methyl]ester ; Fp. 52° ; $[\alpha]_D^{20}$ + 46,87° (c = 0,65 % in $CHCl_3$).

## Beispiel 6

Zu 100 ml 36 %igem Formalin werden bei 10° 100 ml Piperidin unter Kühlung getropft und daraufhin 73 g Acetonoxim sowie 75 g Kaliumcarbonat gegeben. Das Reaktionsgemisch wird über Nacht bei Raumtemperatur gerührt, danach in Aethylacetat aufgenommen und die wässrige Phase abgetrennt. Die organische Phase wird eingedampft, in Aceton aufgenommen und unter Kühlung tropfenweise mit 65 ml Methyljodid versetzt, wonach das Produkt langsam auskristallisiert. Nach weiteren 10 Stunden filtriert man das Produkt ab. Man erhält 291 g N-[(Isopropylidenamino)oxy]-N-methyl-piperidiniumjodid ; Fp. 132-133°.

## Beispiel 7

1,1 g Natriumlactat, 3,5 g N-[(Isopropylidenamino)-oxy]-N-methyl-piperidiniumjodid und 0,1 g 15-Crown-5 in 10 ml Diglykoldimethyläther werden 4 Stunden bei 110° unter Stickstoff erwärmt. Danach wird das Reaktionsgemisch auf 50 ml Wasser gegossen und dreimal mit Aethylacetat extrahiert. Die organische Phase wird viermal mit Wasser gewaschen und anschliessend eingedampft. Nach Chromatographieren des Rückstandes an der 10-fachen Menge Kieselgel mit n-Hexan/Aethylacetat (1 : 1) und Eindampfen des Eluats erhält man 0,35 g L(−)-Milchsäure-/[(isopropylidenamino)oxy]-methyl/ester ; $[\alpha]_D^{20}$ − 1,2° (c = 1,50 % in $CHCl_3$).

# III. Formulierungsbeispiele

## Beispiel 8

Zur Herstellung eines emulgierbaren Konzentrates werden die nachstehend aufgeführten Bestandteile miteinander vermischt :

| | |
|---|---|
| Verbindung der Formel I | 500 g |
| Kondensationsprodukte eines Alkylphenols und Aethylenoxyd /Dodecylbenzolsulfon-saures Calcium | 100 g |

| | |
|---|---|
| Epoxydiertes Soyaöl mit einem Oxiransauerstoffgehalt von ca. 6 % | 25 g |
| Butyliertes Hydroxytoluol | 10 g |

Diese Mischung wird mit Xylol auf 1 Liter aufgefüllt.

## Beispiel 9

Der Wirkstoff, beispielsweise 2-/p-[(α,α,α-Trifluor-p-tolyl)oxy]phenoxy/-propionsäure-/[(isopropylidenamino)-oxy]methyl/ester (Verbindung A in der nachstehenden Tabelle), wird so in Aceton gelöst, dass eine 2 % Lösung an Wirkstoff entsteht. Kurz vor Versuchsbeginn wird mit Wasser auf die gewünschte Konzentration verdünnt, die im vorliegenden Versuch 312 g/ha beträgt. (Bei Verwendung von unlöslichen Wirkstoffen werden Spritzpulver formuliert, die Kaolin als inertes Verdünnungsmittel enthalten).

Die Testpflanzen werden im Gewächshaus mit dem formulierten Wirkstoff besprüht; mittels Quecksilberdampflampen wird ein 16 Stunden Tag simuliert. 3 Wochen nach dem Besprühen werden die Pflanzen auf die eingetretene Wirkung untersucht, d. h. es werden die % Nekrose bestimmt, wobei 100 % Nekrose einer vollständigen Zerstörung der Pflanze entspricht. Lösungsmitteleffekte — soweit vorhanden — werden mittels der « Abbott-Formel » kompensiert. Die Resultate sind in der nachstehenden Tabelle zusammengefasst.

Tabelle 1 (% Nekrose)

| Wirkstoff | Dosierung g/ha | Alipecurus myosuroides | Digitaria floridana | Avena fatua |
|---|---|---|---|---|
| Verbindung A | 312 | 100 | 100 | 100 |

A = 2-/p-[(α,α,α-Trifluor-p-tolyl)oxy]phenoxy/-propionsäure-/[(isopropylidenamino)oxy]methyl/ester

## Beispiel 10

Tabelle II

% Necrosis bei Baumwolle cv. Stoneville 7A

| Dosierung kg/ha | Verbindung A | Verbindung H |
|---|---|---|
| 1,25 | 0 | 22 |
| 0,62 | 0 | |

Formulierung

| | |
|---|---|
| Wirkstoff | 250 g/l |
| NMP | 300 g/l |
| Tensiofix B 7425® | 100 g/l |
| Phenylsulfonat CA® | 25 g/l |
| Shellsol AB® auf | 1 000 ml |

auffüllen.

Netzmittel : 0,08 % Nonoxynol
Wachstumsstadium bei Behandlung : 2 Blattstadium
Sprühvolumen : 1 000 l/ha
Bonitierung : 10 Tage nach Behandlung
Ort : Gewächsaus

Verbindung A siehe Beispiel 9
Verbindung H = D-2-[p-(p-Trifluormethylphenoxy)phenoxy]-propionsäure-methylester

## Beispiel 11

Tabelle III

% Necrosis bei Soyabohnen cv. Hood

| Dosierung kg/ha | Verbindung A |
|---|---|
| 1,25 | 0 |
| 0,62 | 0 |

Formulierung

| | |
|---|---|
| Wirkstoff | 250 g/l |
| NMP | 300 g/l |
| Tensiofix B 7425® | 100 g/l |
| Phenylsulfonat CA® | 25 g/l |
| Shellsol AB® auf | 1 000 ml |

auffüllen.

Netzmittel : 0,08 % Nonoxynol
Wachstumsstadium bei behandlung : 2 dreiteilige Blätter
Sprühvolumen : 1 000 l/ha
Bonitierung : 2 Wochen nach Behandlung
Ort : Gewächshaus

Verbindung A siehe Beispiel 9

Die in den obigen beispielen 10 und 11 verwendeten Bezeichnungen NMP, Tensiofix 7425®, Phenylsulfonat CA®, Shellsol AB® und Nonoxynol haben die folgende Bedeutung :

NMP : N-Methyl-2-pyrrolidon

Tensiofix B 7425® : Emulgator bestehend aus 60 Teilen eines Blockpolymerisates aus Aethylenoxyd und Propylenoxyd, 20 Teilen des Calciumsalzes einer verzweigtkettigen Dodecylbenzolsulfonsäure und 20 Teilen eines Lösungsmittelgemisches aus Isobutanol und $C_{10}$-Alkylbenzolen.

Phenylsulfonat CA® : Gemisch aus 70 Teilen des Calciumsalzes einer verzweigtkettigen Dodecylbenzolsulfonsäure und 30 Teilen eines Lösungsmittelgemisches aus Isobutanol und $C_{10}$-Alkylbenzolen.

Shellsol AB® : Lösungsmittel bestehend aus einem Gemisch aus $C_{10}$-Alkylbenzolen.

Nonoxynol : Kondensationsprodukt aus Nonylphenol und Aethylenoxyd.

**Ansprüche** (für die Vertragsstaaten : BE, CH, DE, FR, IT, LI, NL, SE)

1. Verbindungen der allgemeinen Formel

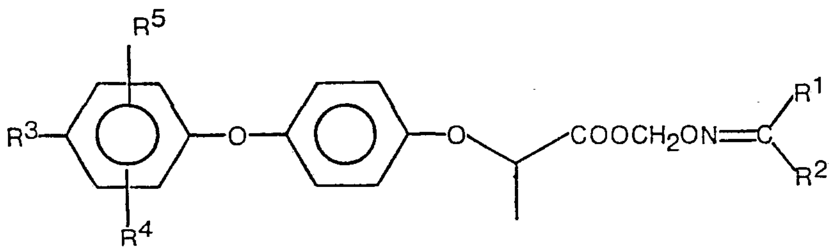

(I)

worin $R^1$ Wasserstoff, Alkyl mit 1-6 Kohlenstoffatomen oder Phenyl, $R^2$ Alkyl mit 1-6 Kohlenstoffatomen oder Phenyl, $R^2$ Alkyl mit 1-6 Kohlenstoffatomen, Alkenyl mit 2-6 Kohlenstoffatomen, Alkinyl mit 2-6 Kohlenstoffatomen oder Phenyl, oder $R^1$ und $R^2$ zusammen mit dem Kohlenstoffatom, an das sie geknüpft sind, einen Cycloalkanring mit 4-10 Kohlenstoffatomen bilden, der gegebenenfalls mit Alkyl mit 1-3 Kohlenstoffatomen mono-, di- oder tri-substituiert sein kann, $R^3$ Halogen, Trifluormethyl oder Nitro und $R^4$ und $R^5$ Wasserstoff oder Halogen darstellen.

2. Verbindungen der allgemeinen Formel I nach Anspruch 1
worin $R^1$ Wasserstoff, Alkyl mit 1-6 Kohlenstoffatomen oder Phenyl, $R^2$ Alkyl mit 1-6 Kohlenstoffatomen, Alkenyl mit 2-6 Kohlenstoffatomen, Alkinyl mit 2-6 Kohlenstoffatomen oder Phenyl, oder $R^1$ und $R^2$ zusammen mit dem Kohlenstoffatom, an das sie geknüpft sind, einen Cycloalkanring mit 4-6 Kohlenstoffatomen bilden, der gegebenenfalls mit Alkyl mit 1-3 Kohlenstoffatomen mono-, di- oder tri-substituiert sein kann, $R^3$ Halogen, Trifluormethyl oder Nitro und $R^4$ und $R^5$ Wasserstoff oder Halogen darstellen.

3. Verbindungen nach Anspruch 2, worin $R^1$ Methyl oder Aethyl bedeutet.

4. Verbindungen nach Anspruch 2 oder 3, worin $R^2$ Methyl, Aethyl oder Phenyl bedeutet.

5. Verbindungen nach einem der Ansprüche 2 bis 4, worin $R^3$ Chlor oder Trifluormethyl bedeutet.

6. Verbindungen nach einem der Ansprüche 2 bis 5, worin $R^4$ Wasserstoff bedeutet.

7. Verbindungen nach einem der Ansprüche 2 bis 6, worin $R^5$ Wasserstoff bedeutet.

8. 2-/p-[(α,α,α-Trifluor-p-tolyl)oxy]phenoxy/-propionsäure-/[(isopropylidenamino)oxy]methyl/ester.

9. 2-[p-(p-Chlorphenoxy)phenoxy]-propionsäure-/[(isopropylidenamino)oxy]methyl/ester.

10. Eine Verbindung nach Anspruch 2, ausgewählt unter :
D-2-/p-[(α,α,α-Trifluor-p-tolyl)oxy]phenoxy/-propionsäure-/[(isopropylidenamino)oxylmethyl/ester,
D-2-/p-[(α,α,α-Trifluor-p-tolyl)oxy]phenoxy/-propionsäure-/[(α-methyl-benzylidenamino)oxy]methyl/ester,

D-2-[p-(p-Chlorphenoxy)phenoxy]-propionsäure-/[(isopropylidenamino)oxy]methyl/ester,
2-[p-(o,p-Dichlorphenoxy)phenoxy]-propionsäure-/[(isopropylidenamino)oxy]methyl/ester,
2-[p-(o,p-Dichlorphenoxy)phenoxy]-propionsäure-/[(α-methyl-benzylidenamino)oxy]methyl/ester,
D-2-[p-(p-Bromphenoxy)phenoxy]-propionsäure-/[(isopropylidenamino)oxy]methyl/ester,
D-2-/p-[(α,α,α-Trifluor-p-tolyl)oxy]phenoxy/-propionsäure-/[(cyclopentylidenamino)oxy]methyl/ester,
D-2-/p-[(α,α,α-Trifluor-p-tolyl)oxy]phenoxy/-propionsäure-/[(cyclohexylidenamino)oxy]methyl/ester,
D-2-/p-[(α,α,α-Trifluor-p-tolyl)oxy]phenoxy/-propionsäure-/[(benzylidenamino)oxy]methyl/ester,
D-2-/p-[(α,α,α-Trifluor-p-tolyl)oxy]phenoxy/-propionsäure-/[(sec. butylidenamino)oxy]methyl/ester und
D-2-/p-[(α,α,α-Trifluor-p-tolyl)oxy]phenoxy/-propionsäure-/[(1-pentyl-hexylidenamino) oxy]methyl/ester.

11. Eine verbindung nach Anspruch 1, ausgewählt unter :
D-2-[p-(p-Jodphenoxy)phenoxy]-propionsäure-/[(Isopropylidenamino)oxy]methyl/ester,
D-2-/p-[(α,α,α-Trifluor-p-tolyl)oxy]phenoxy/-propionsäure-/[(3,3,5-trimethylcyclohexylidenamino)oxy] methyl/ester und
D-2-/p-[(α,α,α-Trifluor-p-tolyl)oxy]phenoxy/-propionsäure-/[1,3-dimethyl-2-butenylidenamino) oxy]methyl/ester.

12. Die D-Isomeren der Verbindungen der Formel I nach Anspruch 1.

13. Die D-Isomeren der Verbindungen der Formel I nach einem der Ansprüche 2 bis 7.

14. Unkrautbekämpfungsmittel, dadurch gekennzeichnet, dass es eine wirksame Menge mindestens einer Verbindung der allgemeinen Formel

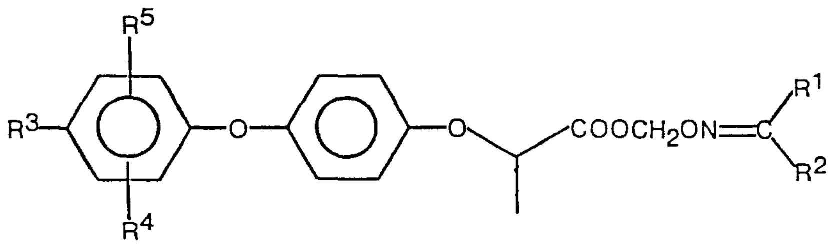

(I)

worin $R^1$ Wasserstoff, Alkyl mit 1-6 Kohlenstoffatomen oder Phenyl, $R^2$ Alkyl mit 1-6 Kohlenstoffatomen, Alkenyl mit 2-6 Kohlenstoffatomen, Alkinyl mit 2-6 Kohlenstoffatomen oder Phenyl, oder $R^1$ und $R^2$ zusammen mit dem Kohlenstoffatom, an das sie geknüpft sind, einen Cycloalkanring mit 4-10 Kohlenstoffatomen bilden, der gegebenenfalls mit Alkyl mit 1-3 Kohlenstoffatomen mono-, di- oder tri-substituiert sein kann, $R^3$ Halogen, Trifluormethyl oder Nitro und $R^4$ und $R^5$ Wasserstoff oder Halogen darstellen, und inertes Trägermaterial enthält.

15. Unkrautbekämpfungsmittel nach Anspruch 14, dadurch gekennzeichnet, daß es eine wirksame Menge mindestens einer Verbindung nach Anspruch 2 und inertes Trägermaterial enthält.

16. Unkrautbekämpfungsmittel nach Anspruch 15, dadurch gekennzeichnet, dass es eine wirksame Menge von 2-/p-[(α,α,α-Trifluor-p-tolyl)oxy]phenoxy/-propionsäure-/[(isopropylidenamino)oxy]methyl/ester enthält.

17. Unkrautbekämpfungsmittel nach Anspruch 15, dadurch gekennzeichnet, dass es eine wirksame Menge von 2-[p-(p-Chlorphenoxy)phenoxy]-propionsäure-/[(isopropylidenamino)-oxy]methyl/ester enthält.

18. Unkrautbekämpfungsmittel nach Anspruch 14, worin als Verbindung bzw. Verbindungen der Formel I die D-Isomeren verwendet werden.

19. Unkrautbekämpfungsmittel nach einem der Ansprüche 15 bis 17, worin als Verbindung bzw. Verbindungen der Formel I die D-Isomeren verwendet werden.

20. Verfahren zur Herstellung von Verbindungen der allgemeinen Formel

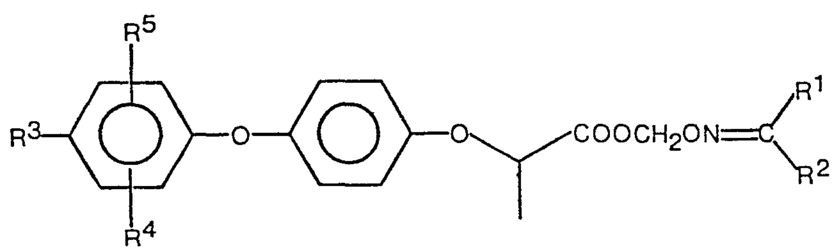

(I)

worin $R^1$ Wasserstoff, Alkyl mit 1-6 Kohlenstoffatomen oder Phenyl, $R^2$ Alkyl mit 1-6 Kohlenstoffatomen, Alkenyl mit 2-6 Kohlenstoffatomen, Alkinyl mit 2-6 Kohlenstoffatomen oder Phenyl, oder $R^1$ und $R^2$ zusammen mit dem Kohlenstoffatom, an das sie geknüpft sind, einen Cycloalkanring mit 4-10 Kohlenstoffatomen bilden, der gegebenenfalls mit Alkyl mit 1-3 Kohlenstoffatomen mono-, di- oder tri-substituiert sein kann, $R^3$ Halogen, Trifluormethyl oder Nitro und $R^4$ und $R^5$ Wasserstoff oder Halogen darstellen, dadurch gekennzeichnet, dass man

a) ein Salz einer Säure der Formel

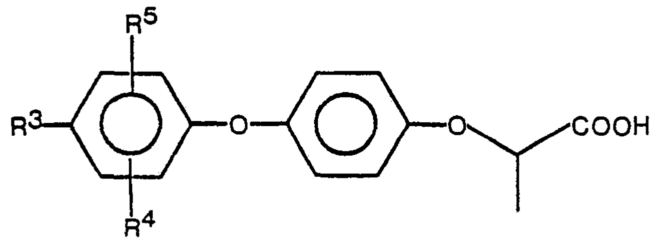

(II)

worin $R^3$, $R^4$ und $R^5$ die für Formel I angegebene Bedeutung besitzen, mit einer Verbindung der Formel

$$R^1R^2CNOCH_2X \quad (III)$$

worin $R^1$ und $R^2$ die für Formel I angegebene Bedeutung besitzen und X eine Abgangsgruppe bedeutet, umsetzt, oder

b) eine Verbindung der Formel

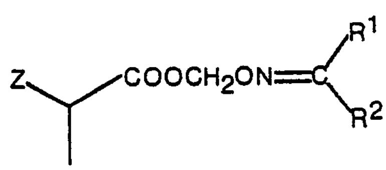

(IV)

worin Z eine Abgangsgruppe bedeutet und $R^1$ und $R^2$ die für Formel I angegebene Bedeutung besitzen, mit einer Verbindung der Formel

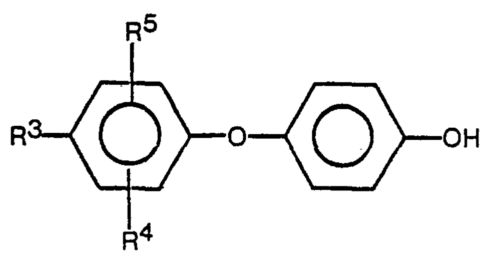

(V)

worin $R^3$, $R^4$ und $R^5$ die für Formel I angegebene Bedeutung besitzen, oder einem Alkalimetallsalz davon umsetzt, oder

c) eine Verbindung der Formel

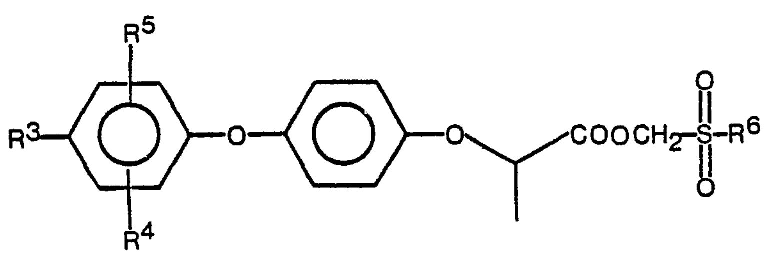

(VI)

worin $R^6$ eine niedere Alkyl-, Aryl- oder Heteroarylgruppe bedeutet und $R^3$, $R^4$ und $R^5$ die für Formel I angegebene Bedeutung besitzen, mit einer Verbindung der Formel

$$R^1R^2CNOH \quad (VII)$$

worin $R^1$ und $R^2$ die für Formel I angegebene Bedeutung besitzen, oder einem Alkalimetallsalz davon in Gegenwart einer Base umsetzt.

21. Verfahren nach Anspruch 20 zur Herstellung von Verbindungen nach Anspruch 2, wobei $R^1$ und

$R^2$ die in Anspruch 2 angegebenen bedeutungen besitzen und X und Z Chlor, Brom, Jod, Mesyloxy oder Tosyloxy bedeuten.

22. Verfahren nach Anspruch 21 zur Herstellung des 2-/p-[(α,α,α-Trifluor-p-tolyl)oxy]phenoxy/-propionsäure/](isopropylidenamino)oxy]methyl/esters, dadurch gekennzeichnet, dass man den 2-/p-[(α,α,α-Trifluor-p-tolyl)oxy]-phenoxy/-propionsäure-[(methylsulfonyl)methyl]ester mit Acetonoximnatriumsalz umsetzt.

23. Verfahren nach Anspruch 20, dadurch gekennzeichnet, dass man das Ausgangsmaterial der Formel VI in D-Form verwendet.

24. Verfahren nach Anspruch 21 oder 22, dadurch gekennzeichnet, dass man das Ausgangsmaterial der Formel VI in D-Form verwendet.

25. Verwendung einer in Anspruch 1, 11 oder 12 genannten Verbindung bzw. eines in Anspruch 14 oder 18 genannten Mittels zur Bekämpfung von Unkräutern.

26. Verwendung einer der in den Ansprüchen 2 bis 10 und 13 genannten Verbindungen bzw. eines in Anspruch 15, 16 oder 17 genannten Mittels zur Bekämpfung von Unkräutern.

**Ansprüche** (für den Vertragsstaat AT)

1. Unkrautbekämpfungsmittel, dadurch gekennzeichnet, dass es eine wirksame Menge mindestens einer Verbindung der allgemeinen Formel

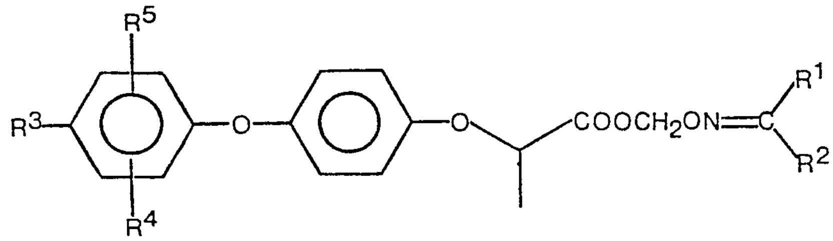

(I)

worin $R^1$ Wasserstoff, Alkyl mit 1-6 Kohlenstoffatomen oder Phenyl, $R^2$ Alkyl mit 1-6 Kohlenstoffatomen, Alkenyl mit 2-6 Kohlenstoffatomen, Alkinyl mit 2-6 Kohlenstoffatomen oder Phenyl, oder $R^1$ und $R^2$ zusammen mit dem Kohlenstoffatom, an das sie geknüpft sind, einen Cycloalkanring mit 4-10 Kohlenstoffatomen bilden, der gegebenenfalls mit Alkyl mit 1-3 Kohlenstoffatomen mono-, di- oder tri-substituiert sein kann, $R^3$ Halogen, Trifluormethyl oder Nitro und $R^4$ und $R^5$ Wasserstoff oder Halogen darstellen, und inertes Trägermaterial enthält.

2. Unkrautbekämpfungsmittel nach Anspruch 1, dadurch gekennzeichnet, daß es eine wirksame Menge mindestens einer Verbindung der allgemeinen Formel I nach Anspruch 1 worin $R^1$ Wasserstoff, Alkyl mit 1-6 Kohlenstoffatomen oder Phenyl, $R^2$ Alkyl mit 1-6 Kohlenstoffatomen, Alkenyl mit 2-6 Kohlenstoffatomen. Alkinyl mit 2-6 Kohlenstoffatomen oder Phenyl, oder $R^1$ und $R^2$ zusammen mit dem Kohlenstoffatom, an das sie geknüpft sind, einen Cycloalkanring mit 4-6 Kohlenstoffatomen bilden, der gegebenenfalls mit Alkyl mit 1-3 Kohlenstoffatomen mono-, di- oder tri-substituiert sein kann, $R^3$ Halogen, Trufluormethyl oder Nitro und $R^4$ und $R^5$ Wasserstoff oder Halogen darstellen, und inertes Trägermaterial enthält.

3. Unkrautbekämpfungsmittel nach Anspruch 2, worin $R^1$ Methyl oder Aethyl bedeutet.

4. Unkrautbekämpfungsmittel nach Anspruch 2 oder 3, worin $R^2$ Methyl, Aethyl oder Phenyl bedeutet.

5. Unkrautbekämpfungsmittel nach einem der Ansprüche 2 bis 4, worin $R^3$ Chlor oder Trifluormethyl bedeutet.

6. Unkrautbekämpfungsmittel nach einem der Ansprüche 2 bis 5, worin $R^4$ Wasserstoff bedeutet.

7. Unkrautbekämpfungsmittel nach einem der Ansprüche 2 bis 6, worin $R^5$ Wasserstoff bedeutet.

8. Unkrautbekämpfungsmittel nach Anspruch 2, dadurch gekennzeichnet, dass es eine wirksame Menge von 2-/p-[(α,α,α-Trifluor-p-tolyl)oxy]phenoxy]-propionsäure-/[(isopropylidenamino)oxy]methyl/ester und inertes Trägermaterial enthält.

9. Unkrautbekämpfungsmittel nach Anspruch 2, dadurch gekennzeichnet, dass es eine wirksame Menge von 2-[p-(p-Chlorphenoxy)phenoxy]-propionsäure-/[(isopropylidenamino)oxy]methyl/ester und inertes Trägermaterial enthält.

10. Unkrautbekämpfungsmittel nach Anspruch 2, dadurch gekennzeichnet, dass es eine wirksame Menge mindestens einer Verbindung ausgewählt unter :

D-2-/p-[(α,α,α-Trifluor-p-tolyl)oxy]phenoxy/-propionsäure-/[(isopropylidenamino)oxy]methyl/ester,
D-2-/p-[(α,α,α-Trifluor-p-tolyl)oxy]phenoxy/-propionsäure-/[(α-methyl-benzylidenamino)oxy]methyl/ester,
D-2-[p-(p-Chlorphenoxy)phenoxy]-propionsäure-[(isopropylidenamino)oxy]methyl/ester,
2-[p-(o,p-Dichlorphenoxy)phenoxy]-propionsäure-/[(isopropylidenamino)oxy]methyl/ester,

2-[p-(o,p-Dichlorphenoxy)phenoxy]-propionsäure-/[(α-methyl-benzylidenamino)oxy]methyl/ester,
D-2-[p-(p-Bromphenoxy)phenoxy]-propionsäure-/[(isopropylidenamino)oxy]methyl/ester,
D-2-/p-[(α,α,α-Trifluor-p-tolyl)oxy]phenoxy/-propionsäure-/[(cyclopentylidenamino)oxy]methyl/ester,
D-2-/p-[(α,α,α-Trifluor-p-tolyl)oxy]phenoxy/-propionsäure/[(cyclohexylidenamino)oxy]methyl/ester,
D-2-/p-[(α,α,α-Trifluor-p-tolyl)oxy]phenoxy/-propionsäure-/[(benzylidenamino)oxy]methyl/ester,
D-2-/p-[(α,α,α-Trifluor-p-tolyl)oxy]phenoxy/-propionsäure-/[(sec. butylidenamino)oxy]methyl/ester
und
D-2-/p-[(α,α,α-Trifluor-p-tolyl)oxy]phenoxy/-propionsäure-/[(1-pentyl-hexylidenamino)oxy]methyl/ester,
und inertes Trägermaterial enthält.

11. Unkrautbekämpfungsmittel nach Anspruch 1, dadurch gekennzeichnet, dass es eine wirksame Menge mindestens einer Verbindung ausgewählt unter:
D-2-[p-(p-Jodphenoxy)phenoxy]-propionsäure-/[(isopropylidenamino)oxy]methyl/ester,
D-2-/p-[(α,α,α-Trifluor-p-tolyl)oxy]phenoxy/-propionsäure-/[(3,3,5-trimethylcyclohexylidenamino)oxy]methyl/-ester und
D-2-/p-[(α,α,α-Trifluor-p-tolyl)oxy]phenoxy/-propionsäure-/[(1,3-dimethyl-2-butenylidenamino)oxy]methyl/ester, und inertes Trägermaterial enthält.

12. Unkrautbekämpfungsmittel nach Anspruch 1, worin als Verbindung bzw. Verbindungen der Formel I die D-Isomeren verwendet werden.

13. Unkrautbekämpfungsmittel nach einem der Ansprüche 2 bis 7, worin als Verbindung bzw. Verbindungen der Formel I die D-Isomeren verwendet werden.

14. Verfahren zur Herstellung von Verbindungen der allgemeinen Formel

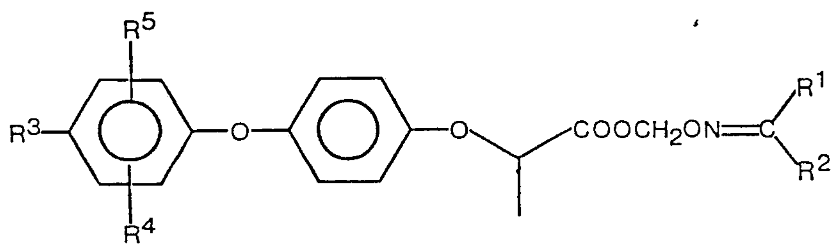

(I)

worin $R^1$ Wasserstoff, Alkyl mit 1-6 Kohlenstoffatomen oder Phenyl, $R^2$ Alkyl mit 1-6 Kohlenstoffatomen, Alkenyl mit 2-6 Kohlenstoffatomen, Alkinyl mit 2-6 Kohlenstoffatomen oder Phenyl, oder $R^1$ und $R^2$ zusammen mit dem Kohlenstoffatom, an das sie geknüpft sind, einen Cycloalkanring mit 4-10 Kohlenstoffatomen bilden, der gegebenenfalls mit Alkyl mit 1-3 Kohlenstoffatomen mono-, di- oder tri-substituiert sein kann, $R^3$ Halogen, Trifluormethyl oder Nitro und $R^4$ und $R^5$ Wasserstoff oder Halogen darstellen, dadurch gekennzeichnet, dass man

a) ein Salz einer Säure der Formel

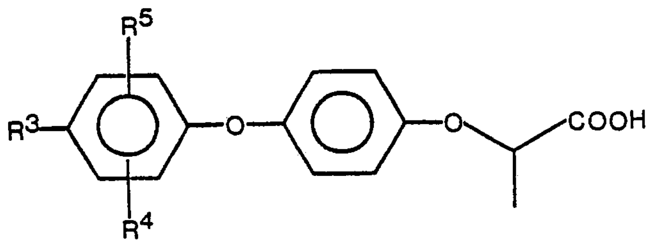

(II)

worin $R^3$, $R^4$ und $R^5$ die für Formel I angegebene Bedeutung besitzen, mit einer Verbindung der Formel

$$R^1R^2CNOCH_2X \qquad \text{(III)}$$

worin $R^1$ und $R^2$ die für Formel I angegebene Bedeutung besitzen und X eine Abgangsgruppe bedeutet, umsetzt, oder

b) eine Verbindung der Formel

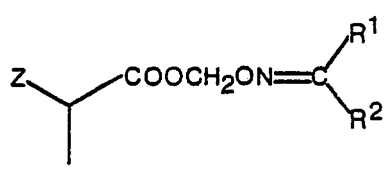

(IV)

worin Z eine Abgangsgruppe bedeutet und $R^1$ und $R^2$ die für Formel I angegebene Bedeutung besitzen, mit einer Verbindung der Formel

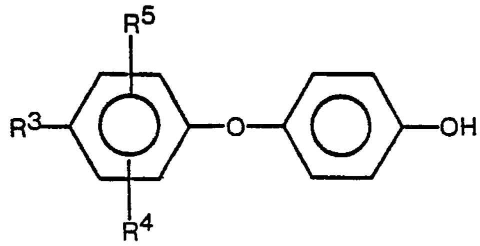

(V)

worin $R^3$, $R^4$ und $R^5$ die für Formel I angegebene Bedeutung besitzen, oder einem Alkalimetallsalz davon umsetzt, oder

c) eine Verbindung der Formel

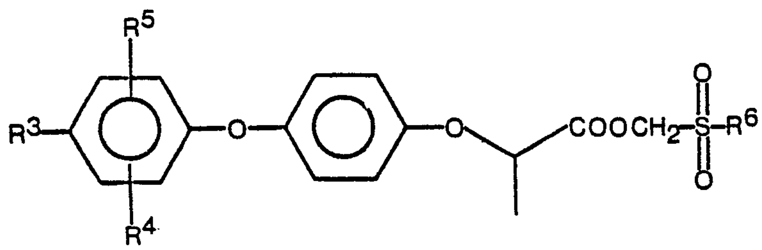

(VI)

worin $R^6$ eine niedere Alkyl-, Aryl- oder Heteroarylgruppe bedeutet und $R^3$, $R^4$ und $R^5$ die für Formel I angegebene Bedeutung besitzen, mit einer Verbindung der Formel

$$R^1R^2CNOH \quad (VII)$$

worin $R^1$ und $R^2$ die für Formel I angegebene Bedeutung besitzen, oder einem Alkalimetallsalz davon in Gegenwart einer Base umsetzt.

15. Verfahren nach Anspruch 14 zur Herstellung von Verbindungen der allgemeinen Formel I nach Anspruch 14 worin $R^1$ Wasserstoff, Alkyl mit 1-6 Kohlenstoffatomen oder Phenyl, $R^2$ Alkyl mit 1-6 Kohlenstoffatomen, Alkenyl mit 2-6 Kohlenstoffatomen, Alkinyl mit 2-6 Kohlenstoffatomen oder Phenyl, oder $R^1$ und $R^2$ zusammen mit dem Kohlenstoffatom, an das sie geknüpft sind, einen Cycloalkanring mit 4-6 Kohlenstoffatomen bilden, der gegebenenfalls mit Alkyl mit 1-3 Kohlenstoffatomen mono-, di- oder trisubstituiert sein kann, $R^3$ Halogen, Trifluormethyl oder Nitro und $R^4$ und $R^5$ Wasserstoff oder Halogen darstellen, und X und Z Chlor, Brom, Jod, Mesyloxy oder Tosyloxy bedeuten.

16. Verfahren nach Anspruch 15 zur Herstellung des 2-/p-[(α,α,α-Trifluor-p-tolyl)oxy]phenoxy/-propionsäure/[(isopropylidenamino)oxy]methyl/esters, dadurch gekennzeichnet, dass man den 2-/p-[(α,α,α-Trifluor-p-tolyl)oxy]-phenoxy/-propionsäure-[(methylsulfonyl)methyl]ester mit Acetonoximnatriumsalz umsetzt.

17. Verfahren nach Anspruch 14 zur Herstellung des 2-[p-(p-Chlorphenoxy)phenoxy]-propionsäure-/[(isopropylidenamino)oxy]methyl/esters, dadurch gekennzeichnet, dass man den 2-[p-(p-chlorphenoxy)phenoxy]-propionsäure-[(methylsulfonyl)methyl]ester mit Acetonoximnatriumsalz umsetzt.

18. Verfahren nach Anspruch 14 oder 17, dadurch gekennzeichnet, dass man das Ausgangsmaterial der Formel VI in D-Form verwendet.

19. Verfahren nach Anspruch 15 oder 16, dadurch gekennzeichnet, dass man das Ausgangsmaterial der Formel VI in D-Form verwendet.

20. Verwendung einer bzw. eines in Anspruch 1, 11 oder 12 genannten Verbindung bzw. Mittels zur Bekämpfung von Unkräutern.

21. Verwendung einer bzw. eines der in den Ansprüchen 2 bis 10 und 13 genannten Verbindungen bzw. Mittel zur Bekämpfung von Unkräutern.

**Claims** (for the Contracting States : BE, CH, DE, FR, IT, LI, NL, SE)

1. Compounds of the general formula

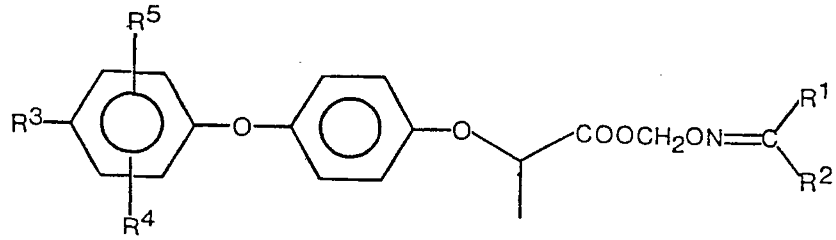

(I)

wherein $R^1$ represents hydrogen, alkyl with 1-6 carbon atoms or phenyl, $R^2$ represents alkyl with 1-6 carbon atoms, alkenyl with 2-6 carbon atoms, alkynyl with 2-6 carbon atoms or phenyl, or $R^1$ and $R^2$ together with the carbon atom to which they are attached form a cycloalkane ring with 4-10 carbon atoms which, if desired, can be mono-, di- or trisubstituted with alkyl with 1-3 carbon atoms, $R^3$ represents halogen, trifluoromethyl or nitro and $R^4$ and $R^5$ represent hydrogen or halogen.

2. Compounds of general formula I according to claim 1, wherein $R^1$ represents hydrogen, alkyl with 1-6 carbon atoms or phenyl, $R^2$ represents alkyl with 1-6 carbon atoms, alkenyl with 2-6 carbon atoms, alkynyl with 2-6 carbon atoms or phenyl, or $R^1$ and $R^2$ together with the carbon atom to which they are attached form a cycloalkane ring with 4-6 carbon atoms which, if desired, can be mono-, di- or trisubstituted with alkyl with 1-3 carbon atoms, $R^3$ represents halogen, trifluoromethyl or nitro and $R^4$ and $R^5$ represent hydrogen or halogen.

3. Compounds according to claim 2, wherein $R^1$ signifies methyl or ethyl.

4. Compounds according to claim 2 or 3, wherein $R^2$ signifies methyl, ethyl or phenyl.

5. Compounds according to any one of claims 2 to 4 wherein $R^3$ signifies chlorine or trifluoromethyl.

6. Compounds according to any one of claims 2 to 5, wherein $R^4$ signifies hydrogen.

7. Compounds according to any one of claims 2 to 6, wherein $R^5$ signifies hydrogen.

8. 2-/p-[(α,α,α-trifluoro-p-tolyl)oxy]phenoxy/-propionic acid/[(isopropylideneamino)oxy]methyl/ester.

9. 2-[p-(p-Chlorophenoxy)phenoxy]-propionic acid/[(isopropylideneamino)oxy]methyl/ester.

10. A compound according to claim 2, selected among :

D-2-/p-[(α,α,α-trifluoro-p-tolyl)oxy]phenoxy/-propionic acid/[(isopropylideneamino)oxy]methyl/ester,

D-2-/p-[(α,α,α-trifluoro-p-tolyl)oxy]phenoxy/-propionic acid/[(α-methyl-benzylideneamino)oxy]methyl/-ester,

D-2-[p-(p-chlorophenoxy)phenoxy]-propionic acid/[(isopropylideneamino)oxy]methyl/ester,

2-[p-(o,p-dichlorophenoxy)phenoxy]-propionic acid/[(isopropulideneamino)oxy]methyl/ester,

2-[p-(o,p-dichlorophenoxy)phenoxy]-propionic acid/[(α-methyl-benzylideneamino)oxy]methyl/ester,

D-2-[p-(p-bromophenoxy)phenoxy]-propionic acid/[(isopropylideneamino)oxy]methyl/ester,

D-2-/p-[(α,α,α-trifluoro-p-tolyl)oxy]phenoxy/-propionic acid/[(cyclopentylideneamino)oxy]methyl/ester,

D-2-/p-[(α,α,α-trifluoro-p-tolyl)oxy]phenoxy/-propionic acid/[(cyclohexylideneamino)oxy]methyl/ester,

D-2-/p-[(α,α,α-trifluoro-p-tolyl)oxy]phenoxy/-propionic acid/[(benzylideneamino)oxy]methyl/ester,

D-2-/p-[(α,α,α-trifluoro-p-tolyl)oxy]phenoxy/-propionic acid/[(sec. butylideneamino)oxy]methyl/ester and

D-2-/p-[(α,α,α-trifluoro-p-tolyl)oxy]phenoxy/-propionic acid/[(1-pentyl-hexylideneamino)oxy] methyl/-ester.

11. A compound according to claim 1, selected among :

D-2-[p-(p-Iodophenoxy)phenoxy]-propionic acid/[(isopropylideneamino)oxy]methyl/ester,

D-2-/p-[(α,α,α-trifluoro-p-tolyl)oxy]phenoxy/-propionic acid/[(3,3,5-trimethylcyclohexylideneamino)oxy]-methyl/ester and

D-2-/p-[(α,α,α-trifluoro-p-tolyl)oxy]phenoxy/-propionic acid/[(1,3-dimethyl-2-butenylideneamino)oxy]-methyl/ester.

12. The D-isomers of the compounds of formula I according to claim 1.

13. The D-isomers of the compounds of formula I according to any one of claims 2 to 7.

14. Weed control composition, characterized in that it contains an effective amount of at least one compound of the general formula

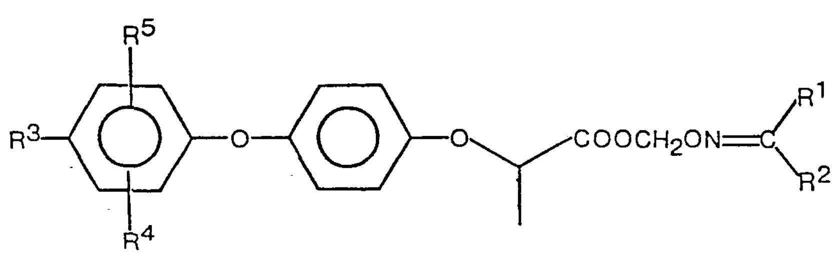

(I)

wherein $R^1$ represents hydrogen, alkyl with 1-6 carbon atoms or phenyl, $R^2$ represents alkyl with 1-6 carbon atoms, alkenyl with 2-6 carbon atoms, alkynyl with 2-6 carbon atoms or phenyl, or $R^1$ and $R^2$

together with the carbon atom to which they are attached represent a cycloalkane ring with 4-10 carbon atoms which, if desired, can be mono-, di- or tri-substituted with alkyl with 1-3 carbon atoms, $R^3$ represents halogen, trifluoromethyl or nitro and $R^4$ and $R^5$ represent hydrogen or halogen, and inert carrier material.

15. Weed control composition according to claim 14, characterized in that it contains an effective amount of at least one compound according to claim 2 and inert carrier material.

16. Weed control composition according to claim 15, characterized in that it contains an effective amount of 2-/p-[(α,α,α-trifluoro-p-tolyl)oxy]phenoxy/-propionic acid/[(isopropylideneamino)oxy]methyl/ester.

17. Weed control composition according to claim 15, characterized in that it contains an effective amount of 2-[p-(p-chlorophenoxy)phenoxy]-propionic acid/[(isopropylideneamino)oxy]methyl/ester.

18. Weed control composition according to claim 14, wherein as the compound or compounds of formula I there are used the D-isomers.

19. Weed control composition according to any one of claims 15 to 17, wherein as the compound or compounds of formula I there are used the D-isomers.

20. Process for the manufacture of compounds of the general formula

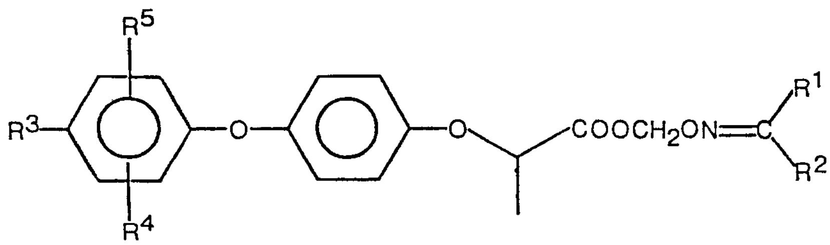

(I)

wherein $R^1$ represents hydrogen, alkyl with 1-6 carbon atoms or phenyl, $R^2$ represents alkyl with 1-6 carbon atoms, alkenyl with 2-6 carbon atoms, alkynyl with 2-6 carbon atoms or phenyl, or $R^1$ and $R^2$ together with the carbon atom to which they are attached form a cycloalkane ring with 4-10 carbon atoms which, if desired, can be mono-, di- or tri-substituted with alkyl with 1-3 carbon atoms, $R^3$ represents halogen, trifluoromethyl or nitro and $R^4$ and $R^5$ represent hydrogen or halogen, characterized by

a) reacting a salt of an acid of the formula

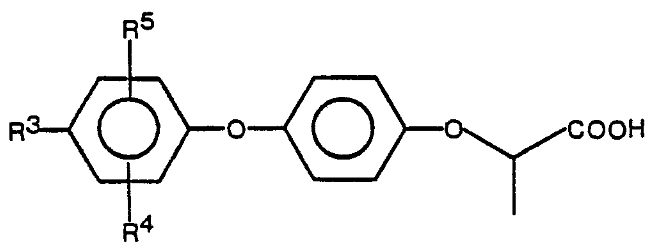

(II)

wherein $R^3$, $R^4$ and $R^5$ have the significance given for formula I, with a compound of the formula

$$R^1R^2CNOCH_2X \quad (III)$$

wherein $R^1$ and $R^2$ have the significance given for formula I and X signifies a leaving group, or

b) reacting a compound of the formula

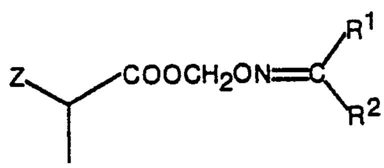

(IV)

wherein Z signifies a leaving group and $R^1$ and $R^2$ have the significance given for formula I, with a compound of the formula

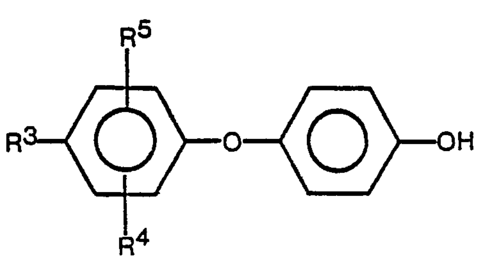

(V)

wherein $R^3$, $R^4$ and $R^5$ have the significance given for formula I, or an alkali metal salt thereof, or

c) reacting a compound of the formula

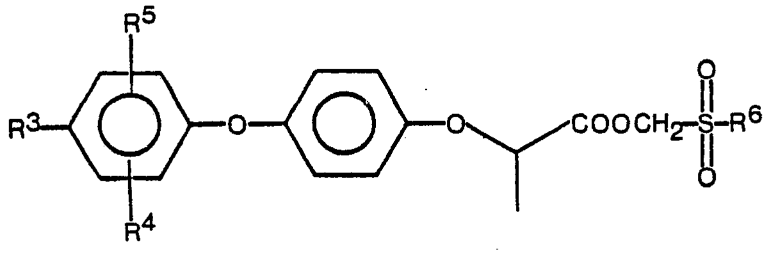

(VI)

wherein $R^6$ signifies a lower alkyl, aryl or heteroaryl group and $R^3$, $R^4$ and $R^5$ have the significance given for formula I, with a compound of the formula

$$R^1R^2CNOH \qquad (VII)$$

wherein $R^1$ and $R^2$ have the significance given for formula I, or an alkali metal salt thereof in the presence of a base.

21. Process according to claim 20 for the manufacture of compounds according to claim 2, whereby $R^1$ and $R^2$ have the significances given in claim 2 and X and Z signify chlorine, bromine, iodine, mesyloxy or tosyloxy.

22. Process according to claim 21 for the manufacture of 2-/p-[(α,α,α-trifluoro-p-tolyl)oxy]phenoxy/-propionic acid/[(isopropylideneamino)oxy]methyl/ester, characterized by reacting 2-/p-[(α,α,α-trifluoro-p-tolyl)oxy]phenoxy/-propionic acid[(methylsulphonyl)methyl]ester with acetone oxime sodium salt.

23. Process according to claim 20, characterized in that the starting material of formula VI is used in D-form.

24. Process according to claim 21 or 22, characterized in that the starting material of formula VI is used in D-form.

25. Use of a compound set forth in claim 1, 11 or 12 or of a composition set forth in claim 14 or 18 for the control of weeds.

26. Use of one of the compounds set forth in claims 2 to 10 and 13 or of a composition set forth in claim 15, 16 or 17 for the control of weeds.

**Claims** (for the Contracting State AT)

1. Weed control composition, characterized in that it contains an effective amount of at least one compound of the general formula

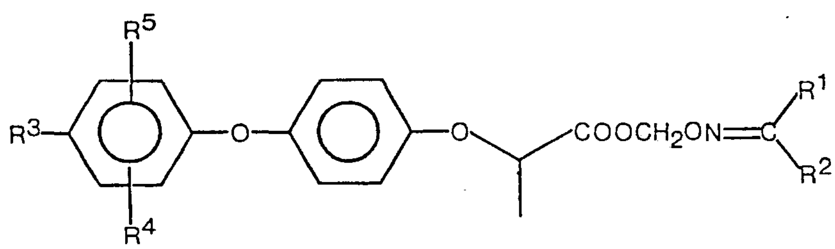

(I)

wherein $R^1$ represents hydrogen, alkyl with 1-6 carbon atoms or phenyl, $R^2$ represents alkyl with 1-6 carbon atoms, alkenyl with 2-6 carbon atoms, alkynyl with 2-6 carbon atoms or phenyl, or $R^1$ and $R^2$ together with the carbon atom to which they are attached form a cycloalkane ring with 4-10 carbon atoms which, if desired, can be mono-, di- or tri-substituted with alkyl with 1-3 carbon atoms, $R^3$ represents halogen, trifluoromethyl or nitro and $R^4$ and $R^5$ represent hydrogen or halogen, and inert carrier material.

2. Weed control composition according to claim 1 characterized in that it contains an effective amount of at least one compound of general formula I according to claim 1, wherein $R^1$ represents hydrogen, alkyl with 1-6 carbon atoms or phenyl, $R^2$ represents alkyl with 1-6 carbon atoms, alkenyl with 2-6 carbon atoms, alkynyl with 2-6 carbon atoms or phenyl, or $R^1$ and $R^2$ together with the carbon atom to which they are attached form a cycloalkane ring with 4-6 carbon atoms which, if desired, can be mono-, di- or tri-substituted with alkyl with 1-3 carbon atoms, $R^3$ represents halogen, trifluoromethyl or nitro and $R^4$ and $R^5$ represent hydrogen or halogen, and inert carrier material.

3. Weed control composition according to claim 2, wherein $R^1$ signifies methyl or ethyl.

4. Weed control composition according to claim 2 or 3, wherein $R^2$ signifies methyl, ethyl or phenyl.

5. Weed control composition according to any one of claims 2 to 4, wherein $R^3$ signifies chlorine or trifluoromethyl.

6. Weed control composition according to any one of claims 2 to 5, wherein $R^4$ signifies hydrogen.

7. Weed control composition according to any one of claims 2 to 6, wherein $R^5$ signifies hydrogen.

8. Weed control composition according to claim 2, characterized in that it contains an effective amount of 2-/p-[(α,α,α-trifluoro-p-tolyl)oxy]phenoxy/-propionic acid/[(isopropylideneamino)oxy]methyl/ester and inert carrier material.

9. Weed control composition according to claim 2, characterized in that it contains an effective amount of 2-[p-(p-chlorophenoxy)phenoxy]-propionic acid/[(isopropylideneamino)oxy]methyl/ester and inert carrier material.

10. Weed control composition according to claim 2, characterized in that it contains an effective amount of at least one compound selected among :

D-2-/p-[(α,α,α-trifluoro-p-tolyl)oxy]phenoxy/-propionic acid/[(isopropylideneamino)oxy]methyl/ester,
D-2-/p-[(α,α,α-trifluoro-p-tolyl)oxy]phenoxy/-propionic acid/[(α-methyl-benzylideneamino)oxy]methyl/ester,
D-2-[p-(p-chlorophenoxy)phenoxy]-propionic acid/[(isopropylideneamino)oxy]methyl/ester,
2-[p-(o,p-dichlorophenoxy)phenoxy]-propionic acid/[(isopropylideneamino)oxy]methyl/ester,
2-[p-(o,p-dichlorophenoxy)phenoxy]-propionic acid/[(α-methyl-benzylideneamino)oxy]methyl/ester,
D-2-[p-(p-bromophenoxy)phenoxy]-propionic acid/[(isopropylideneamino)oxy]methyl/ester,
D-2-/p-[(α,α,α-trifluoro-p-tolyl)oxy]phenoxy/-propionic acid/[(cyclopentylideneamino)oxy]methyl/ester,
D-2-/p-[(α,α,α-trifluoro-p-tolyl)oxy]phenoxy/-propionic acid/[(cyclohexylideneamino)oxy]methyl/ester,
D-2-/p-[(α,α,α-trifluoro-p-tolyl)oxy]phenoxy/-propionic acid/[(benzylideneamino)oxy]methyl/ester,
D-2-/p-[(α,α,α-trifluoro-p-tolyl)oxy]phenoxy/-propionic acid/[(sec. butylideneamino)oxy]methyl/ester and
D-2-/p-[(α,α,α-trifluoro-p-tolyl)oxy]phenoxy/-propionic acid/[(1-pentyl-hexylideneamino)oxy]methyl/ester, and inert carrier material.

11. Weed control composition according to claim 1, characterized in that it contains an effective amount of at least one compound selected among :

D-2-[p-(p-iodophenoxy)phenoxy]-propionic acid/[(isopropylideneamino)oxy]methyl/ester,
D-2-/p-[(α,α,α-trifluoro-p-tolyl)oxy]phenoxy/-propionic acid/[(3,3,5-trimethylcyclohexylideneamino)oxy]methyl/ester and
D-2-/p-[(α,α,α-trifluoro-p-tolyl)oxy]phenoxy/-propionic acid/[(1,3-dimethyl-2-butenylideneamino)oxy]-methyl/ester, and inert carrier material.

12. Weed control composition according to claim 1, wherein as the compound or compounds of formula I there are used the D-isomers.

13. Weed control composition according to any one of claims 2 to 7, wherein as the compound or compounds of formula I there are used the D-isomers.

14. Process for the manufacture of compounds of the general formula

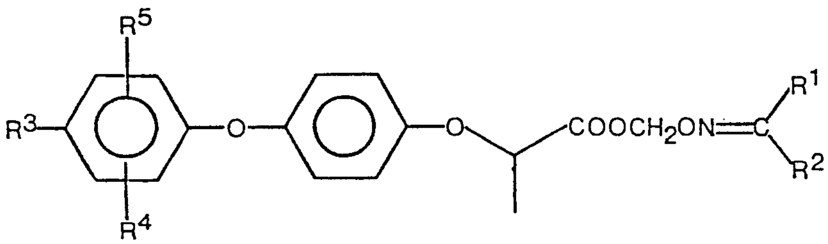

(I)

wherein $R^1$ represents hydrogen, alkyl with 1-6 carbon atoms or phenyl, $R^2$ represents alkyl with 1-6 carbon atoms, alkenyl with 2-6 carbon atoms, alkynyl with 2-6 carbon atoms or phenyl, or $R^1$ and $R^2$ together with the carbon atom to which they are attached form a cycloalkane ring with 4-10 carbon atoms which, if desired, can be mono-, di- or tri-substituted with alkyl with 1-3 carbon atoms, $R^3$ represents halogen, trifluoromethyl or nitro and $R^4$ and $R^5$ represent hydrogen or halogen, characterized by

a) reacting a salt of an acid of the formula

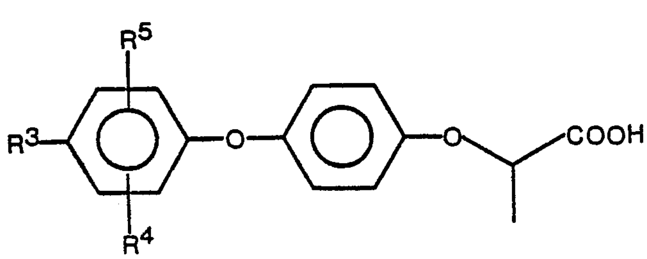

(II)

wherein $R^3$, $R^4$ and $R^5$ have the significance given for formula I, with a compound of the formula

$$R^1R^2CNOCH_2X \qquad (III)$$

wherein $R^1$ and $R^2$ have the significance given for formula I and X signifies a leaving group, or

b) reacting a compound of the formula

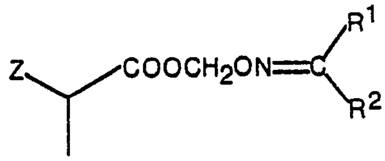

(IV)

wherein Z signifies a leaving group and $R^1$ and $R^2$ have the significance given for formula I, with a compound of the formula

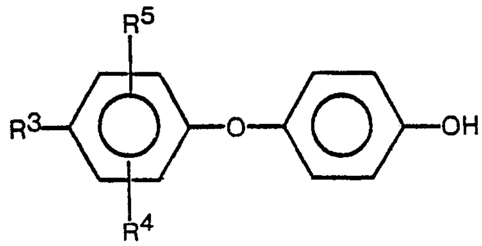

(V)

wherein $R^3$, $R^4$ and $R^5$ have the significance given for formula I, or an alkali metal salt thereof, or

c) reacting a compound of the formula

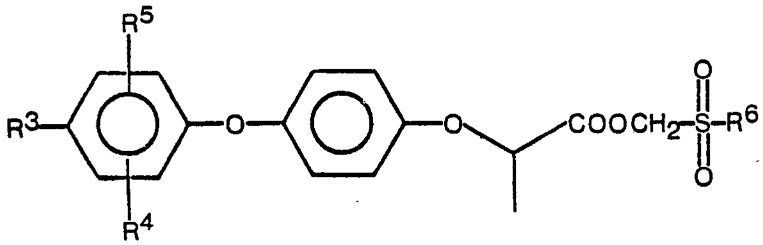

(VI)

wherein $R^6$ signifies a lower alkyl, aryl or heteroaryl group and $R^3$, $R^4$ and $R^5$ have the significance given for formula I, with a compound of the formula

$$R^1R^2CNOH \qquad (VII)$$

wherein $R^1$ and $R^2$ have the significance given for formula I, or an alkali metal salt thereof in the presence of a base.

15. Process according to claim 14 for the manufacture of compounds of general formula I according to claim 14, wherein $R^1$ represents hydrogen, alkyl with 1-6 carbon atoms or phenyl, $R^2$ represents alkyl with 1-6 carbon atoms, alkenyl with 2-6 carbon atoms, alkynyl with 2-6 carbon atoms or phenyl, or $R^1$ and $R^2$ together with the carbon atom to which they are attached form a cycloalkane ring with 4-6 carbon atoms which, if desired, can be mono-, di- or tri-substituted with alkyl with 1-3 carbon, $R^3$ represents halogen, trifluoromethyl or nitro and $R^4$ and $R^5$ represent hydrogen or halogen, and X and Z signify chlorine, bromine, iodine mesyloxy or tosyloxy.

16. Process according to claim 15 for the manufacture of 2-/p-[(α,α,α-trifluoro-p-tolyl)oxy]phenoxy/-propionic acid/[(isopropylideneamino)oxy]methyl/ester, characterized by reacting 2-/p-[(α,α,α-trifluoro-p-tolyl)oxy]phenoxy/-propionic acid[(methylsulphonyl)methyl]ester with acetone oxime sodium salt.

17. Process according to claim 14 for the manufacture of 2-[p-(p-chlorophenoxy)phenoxy]-propionic acid/[(isopropylideneamino)oxy]methyl/ester, characterized by reacting 2-[p-(p-chlorophenoxy)phenoxy]-propionic acid[(methyl-sulphonyl)methyl]ester with acetone oxime sodium salt.

18. Process according to claim 14 or 17, characterized in that the starting material of formula VI is used in D-form.

19. Process according to claim 15 or 16, characterized in that the starting material of formula VI is used in D-form.

20. Use of a compound or composition set forth in claim 1, 11 or 12 for the control of weeds.

21. Use of one of the compounds or compositions set forth in claims 2 to 10 and 13 for the control of weeds.

**Revendications** (pour les Etats contractants : BE, CH, DE, FR, IT, LI, NL, SE)

1. Composés de formule générale

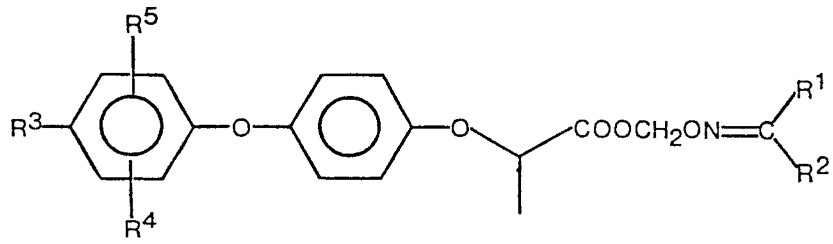

(I)

où $R^1$ représente un hydrogène, un alcoyle en $C_1$ à $C_6$ ou un phényle, $R^2$ représente un alcoyle en $C_1$ à $C_6$, un alcényle en $C_2$ à $C_6$, un alcynyle en $C_2$ à $C_6$ ou un phényle, ou bien où $R^1$ et $R^2$ forment ensemble avec l'atome de carbone auquel ils sont liés un noyau cycloalcane en $C_4$ à $C_{10}$, qui peut éventuellement être mono-, di- ou trisubstitué par un alcoyle en $C_1$ à $C_3$, $R^3$ représente un halogène, un trifluorométhyle ou un nitro et $R^4$ et $R^5$ représentent un hydrogène ou un halogène.

2. Composés de formule générale I selon la revendication 1 où $R^1$ représente un hydrogène, un alcoyle en $C_1$ à $C_6$ ou un phényle, $R^2$ représente un alcoyle en $C_1$ à $C_6$, un alcényle en $C_2$ à $C_6$, un alcynyle en $C_2$ à $C_6$ ou un phényle, ou bien où $R^1$ et $R^2$ forment ensemble avec l'atome de carbone auquel ils sont liés un noyau cycloalcane en $C_4$ à $C_6$, qui peut être éventuellement mono-, di- ou trisubstitué par un alcoyle en $C_1$ à $C_3$, $R^3$ représente un halogène, un trifluorométhyle ou un nitro et $R^4$ et $R^5$ représentent un hydrogène ou un halogène.

3. Composés selon la revendication 2, où $R^1$ représente un méthyle ou un éthyle.

4. Composés selon l'une des revendications 2 ou 3, où $R^2$ représente un méthyle, un éthyle ou un phényle.

5. Composés selon l'une des revendications 2 à 4, où $R^3$ représente un chlore ou un trifluorométhyle.

6. Composés selon l'une des revendications 2 à 5, où $R^4$ représente un hydrogène.

7. Composés selon l'une des revendications 2 à 6, où $R^5$ représente un hydrogène.

8. /[(isopropylidèneamino)oxy]méthyl/ester de l'acide 2-/p-[(α,α,α-trifluoro-p-tolyl)oxy]phénoxy/-propionique.

9. /[(isopropylidèneamino)oxy]méthyl/ester de l'acide 2-[p-(p-chlorophénoxy)phénoxy]-propionique.

10. Composé selon la revendication 2, choisi parmi :

/[(isopropylidèneamino)oxy]méthyl/ester de l'acide D-2-/p-[(α,α,α-trifluoro-p-tolyl)oxy]phénoxy/-propionique,

/[(α-méthyl-benzylidèneamino)oxy]méthyl/ester de l'acide D-2-/p-[(α,α,α-trifluoro-p-tolyl)oxy]phénoxy/-propionique,

/[(isopropylidèneamino)oxy]méthyl/ester de l'acide D-2-[p-(p-chlorophénoxy)phénoxy]-propionique,

/[(isopropylidèneamino)oxy]méthyl/ester de l'acide 2-[p-(o,p-dichlorophénoxy)phénoxy]-propionique,

/[(α-méthyl-benzylidèneamino)oxy]méthyl/ester de l'acide 2-[p-(o,p-dichlorophénoxy)phénoxy]-propionique,

/[(isopropylidèneamino)oxy]méthyl/ester de l'acide D-2-]p-(p-bromophénoxy)phénoxy]-propionique,

/[(cyclopentylidèneamino)oxy]méthyl/ester de l'acide D-2-/p-[(α,α,α-trifluoro-p-tolyl)oxy]phénoxy/-propionique,

/[(cyclohexylidèneamino)oxy]méthyl/ester de l'acide D-2-/p-[(α,α,α-trifluoro-p-tolyl)oxy]phénoxy/-propionique,

/[(benzylidèneamino)oxy]méthyl/ester de l'acide D-2-/p-[(α,α,α-trifluoro-p-tolyloxy)oxy]phénoxy/-propionique,

/[(sec-butylidèneamino)oxy]méthyl/ester de l'acide D-2-/p-[(α,α,α-trifluoro-p-tolyl)oxy]phénoxy/-propionique et

/[(1-pentyl-hexylidèneamino)oxy]méthyl/ester de l'acide D-2-/p-[(α,α,α-trifluoro-p-tolyl)oxy]phénoxy/-propionique.

11. Composé selon la revendication 1, choisi parmi :

/[isopropylidèneamino)oxy]méthyl/ester de l'acide D-2-[p-(p-iodo-)phénoxy)phénoxy]-propionique,

/[(3,3,5-triméthylcyclohexylidèneamino)oxy]méthyl/ester de l'acide D-2-/p-[(α,α,α-trifluoro-p-tolyl)/-oxy]phénoxy/-propionique et

/[(1,3-diméthyl-2-buténylidèneamino)oxy]méthyl/ester de l'acide D-2-/p-[(α,α,α-trifluoro-p-tolyl)oxyl] phénoxy/-propionique.

12. Les isomères D des composés de formule I selon la revendication 1.

13. Les isomères D des composés de formule I selon l'une des revendications 2 à 7.

14. Agent de lutte contre les mauvaises herbes caractérisé en ce qu'il contient une quantité efficace d'au moins un composé de formule générale

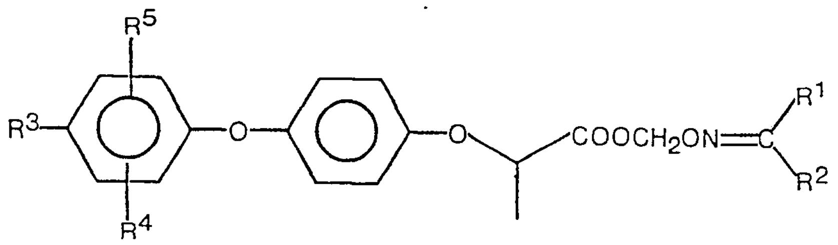

(I)

où $R^1$ représente un hydrogène, un alcoyle en $C_1$ à $C_6$ ou un phényle $R^2$ représente un alcoyle en $C_1$ à $C_6$, un alcényle en $C_2$ à $C_6$, un alcynyle en $C_2$ à $C_6$ ou un phényle, ou bien où $R^1$ et $R^2$ forment ensemble avec l'atome de carbone auquel ils sont liés un noyau cycloalcane en $C_4$ à $C_{10}$ qui peut être éventuellement mono-, di- ou trisubstitué par un alcoyle en $C_1$ à $C_3$, $R^3$ représente un halogène, un trifluorométhyle ou un nitro et $R^4$ et $R^5$ représentent un hydrogène ou un halogène, et un support inerte.

15. Agent de lutte contre les mauvaises herbes, caractérisé en ce qu'il contient une quantité efficace d'au moins un composé selon la revendication 2 et un support inerte.

16. Agent de lutte contre les mauvaises herbes selon la revendication 15, caractérisé en ce qu'il contient une quantité efficace d'/[(isopropylidèneamino)oxy]méthyl/ester de l'acide 2-/p-[(α,α,α-trifluoro-p-tolyl)oxy]phénoxy/-propionique.

17. Agent de lutte contre les mauvaises herbes selon la revendication 15, caractérisé en ce qu'il contient une quantité efficace d'/[(isopropylidèneamino)oxy]méthyl/ester de l'acide 2-[p-(p-chlorophénoxy)phénoxy]-propionique.

18. Agent de lutte contre les mauvaises herbes selon la revendication 14, où l'on utilise comme composé(s) de formule I les isomères D.

19. Agent de lutte contre les mauvaises herbes selon l'une des revendications 15 à 17, où l'on utilise comme composé(s) de formule I les isomères D.

20. Procédé de préparation de composés de formule générale

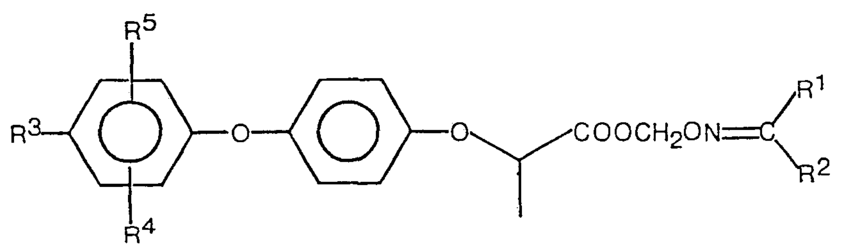

(I)

où $R^1$ représente un hydrogène, un alcoyle en $C_1$ à $C_6$ ou un phényle, $R^2$ représente un alcoyle en $C_1$ à $C_6$, un alcényle en $C_2$ à $C_6$, un alcynyle en $C_2$ à $C_6$ ou un phényle, ou bien où $R^1$ et $R^2$ forment ensemble avec l'atome de carbone auquel ils sont liés, un noyau cycloalcane en $C_4$ à $C_{10}$, qui peut éventuellement être mono-, di- ou trisubstitué par un alcoyle en $C_1$ à $C_3$, $R^3$ représente un halogène, un trifluorométhyle ou un nitro et $R^4$ et $R^5$ représentent un hydrogène ou un halogène, caractérisé en ce que

a) on fait réagir un sel d'un acide de formule

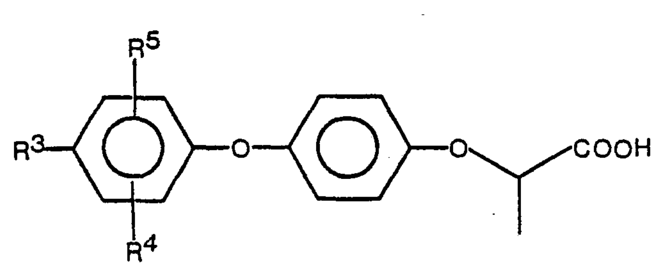

(II)

où $R^3$, $R^4$ et $R^5$ ont la signification donnée pour la formule I, avec un composé de formule

$$R^1R^2CNOCH_2X \qquad (III)$$

où $R^1$ et $R^2$ ont la signification donnée pour la formule I et X représente un groupe sortant, ou

b) on fait réagir un composé de formule

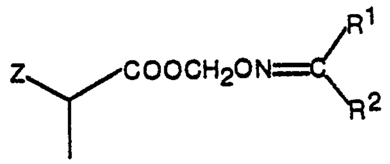

(IV)

où Z représente un groupe sortant et où $R^1$ et $R^2$ ont la signification donnée pour la formule I, avec un composé de formule

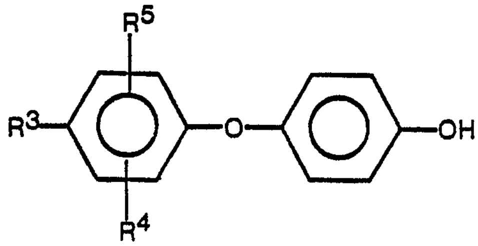

(V)

où $R^3$, $R^4$ et $R^5$ ont la signification donnée pour la formule I, ou un de ses sels de métaux alcalins, ou

c) on fait réagir un composé de formule

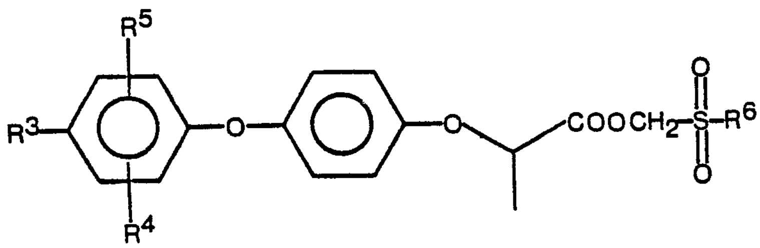

(VI)

où $R^6$ représente un groupe alcoyle inférieur, aryle ou hétéroaryle et où $R^3$, $R^4$ et $R^5$ ont la signification donnée pour la formule I, avec un composé de formule

$$R^1R^2CNOH \quad (VII)$$

où $R^1$ et $R^2$ ont la signification donnée pour la formule I, ou un de ses sels de métaux alcalins en présence d'une base.

21. Procédé selon la revendication 20 de préparation de composés selon la revendication 2, où $R^1$ et $R^2$ ont les significations données dans la revendication 2 et où X et Z représentent un chlore, un brome, un iode, un mésyloxy ou un tosyloxy.

22. Procédé selon la revendication 21 de préparation de l'/[(isopropylidèneamino)oxy]méthyl/ester de l'acide 2-/p-[(α,α,α-trifluoro-p-tolyl)oxy]-phénoxy/-propionique, caractérisé en ce qu'on fait réagir le [(méthylsulfonyl)méthyl]-ester de l'acide 2-/p-[(α,α,α-trifluoro-p-tolyl)oxy]-phénoxy/-propionique avec le sel de sodium d'acétonoxime.

23. Procédé selon la revendication 20 caractérisé en ce qu'on utilise le produit de départ de formule VI sous forme D.

24. Procédé selon l'une des revendications 21 ou 22, caractérisé en ce qu'on utilise le produit de départ de formule VI sous forme D.

25. Application d'un composé mentionné dans la revendication 1, 11 ou 12 ou d'un agent mentionné dans la revendication 14 ou 18 à la lutte contre les mauvaises herbes.

26. Application d'un des composés mentionnés dans les revendications 2 à 10 et 13, ou d'un agent mentionné dans la revendication 15, 16 ou 17 à la lutte contre les mauvaises herbes.

**Revendications** (pour l'Etat contractant AT)

1. Agent de lutte contre les mauvaises herbes, caractérisé en ce qu'il contient une quantité efficace d'au moins un composé de formule générale

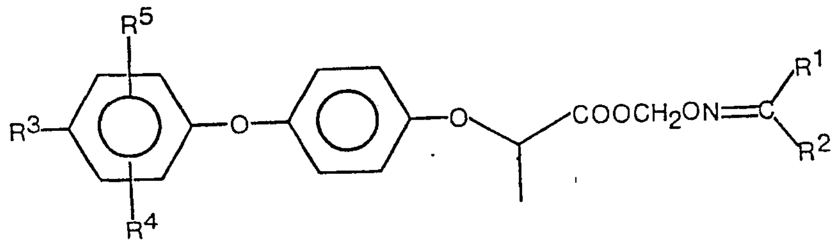

(I)

où $R^1$ représente un hydrogène, un alcoyle en $C_1$ à $C_6$ ou un phényle, $R^2$ représente un alcoyle en $C_1$ à $C_6$, un alcényle en $C_2$ à $C_6$, un alcynyle en $C_2$ à $C_6$ ou un phényle, ou bien où $R^1$ et $R^2$ forment ensemble avec l'atome de carbone auquel ils sont liés un noyau cycloalcane en $C_4$ à $C_{10}$, qui peut éventuellement être mono-, di- ou trisubstitué par un alcoyle en $C_1$ à $C_3$, $R^3$ représente un halogène, un trifluorométhyle ou un nitro et $R^4$ et $R^5$ représentent un hydrogène ou un halogène, et un support inerte.

2. Agent de lutte contre les mauvaises herbes selon la revendication 1, caractérisé en ce qu'il contient une quantité efficace d'au moins un composé de formule générale I selon la revendication 1, où $R^1$ représente un hydrogène, un alcoyle en $C_1$ à $C_6$ ou un phényle, $R^2$ représente un alcoyle en $C_1$ à $C_6$, un alcényle en $C_2$ à $C_6$, un alcynyle en $C_2$ à $C_6$ ou un phényle, ou bien où $R^1$ et $R^2$ forment ensemble avec l'atome de carbone auquel ils sont liés un noyau cycloalcane en $C_4$ à $C_6$, qui peut éventuellement être mono-, di- ou trisubstitué par un alcoyle en $C_1$ à $C_3$, $R^3$ représente un halogène, un trifluorométhyle ou un nitro et $R^4$ et $R^5$ représentent un hydrogène ou un halogène, et un support inerte.

3. Agent de lutte contre les mauvaises herbes selon la revendication 2, où $R^1$ représente un méthyle ou un éthyle.

4. Agent de lutte contre les mauvaises herbes selon l'une des revendications 2 ou 3, où $R^2$ représente un méthyle, un éthyle ou un phényle.

5. Agent de lutte contre les mauvaises herbes selon l'une des revendications 2 à 4, où $R^3$ représente un chlore ou un trifluorométhyle.

6. Agent de lutte contre les mauvaises herbes selon l'une des revendications 2 à 5, où $R^4$ représente un hydrogène.

7. Agent de lutte contre les mauvaises herbes selon l'une des revendications 2 à 6, où $R^5$ représente un hydrogène.

8. Agent de lutte contre les mauvaises herbes selon la revendication 2, caractérisé en ce qu'il contient une quantité efficace d'/[(isopropylidèneamino)oxy]méthyl/ester de l'acide 2-/p-[(α,α,α-trifluoro-p-tolyl)oxy]phénoxy/propionique et un support inerte.

9. Agent de lutte contre les mauvaises herbes selon la revendication 2, caractérisé en ce qu'il contient une quantité efficace d'/[(isopropylidèneamino)oxy]méthyl/ester de l'acide 2-[p-(p-chlorophénoxy)phénoxy]-propionique et un support inerte.

10. Agent de lutte contre les mauvaises herbes selon la revendication 2, caractérisé en ce qu'il contient une quantité efficace d'au moins un composé choisi parmi :

/[(isopropylidèneamino)oxy]méthyl/ester de l'acide D-2-/p-[(α,α,α-trifluoro-p-tolyl)oxy]phénoxy/-propionique,

/[(α-méthyl-benzylidèneamino)oxy]méthyl/ester de l'acide D-2-/p-[(α,α,α-trifluoro-p-tolyl)oxy]phénoxy/-propionique,

/[(isopropylidèneamino)oxy]méthyl/ester de l'acide D-2-[p-(p-chlorophénoxy)phénoxy]-propionique,

/[(isopropylidèneamino)oxy]méthyl/ester de l'acide 2-[p-(o,p-dichlorophénoxy)phénoxy]-propionique,

/[(α-méthyl-benzylidèneamino)oxy]méthyl/ester de l'acide 2-[p-(o,p-dichlorophénoxy)phénoxy]-propionique,

/[(isopropylidèneamino)oxy]méthyl/ester de l'acide D-2-[(p-(p-bromophénoxy)phénoxy]-propionique,

/[(cyclopentylidèneamino)oxy]méthyl/ester de l'acide D-2-/p-[(α,α,α-trifluoro-p-tolyl)oxy]-phénoxy/-propionique,

/[(cyclohexylidèneamino)oxy]méthyl/ester de l'acide D-2-/p-[(α,α,α-trifluoro-p-tolyl)oxy]phénoxy/-propionique,

/[(benzylidèneamino)oxy]méthyl/ester de l'acide D-2-/p-[(α,α,α-trifluoro-p-tolyl)oxy]-phénoxy/-propionique,

/[(sec. butylidèneamino)oxy]méthyl/ester de l'acide D-2-/p-[(α,α,α-trifluoro-p-tolyl)oxy]phénoxy/-propionique et

/[(1-pentyl-hexylidèneamino)oxy]méthyl/ester de l'acide D-2-/p-[(α,α,α-trifluoro-p-tolyl)oxy]phénoxy/-propionique, et un support inerte.

11. Agent de lutte contre les mauvaises herbes selon la revendication 1, caractérisé en ce qu'il contient une quantité efficace d'au moins un composé choisi parmi :

/[(isopropylidèneamino)oxy]méthyl/ester de l'acide D-2-[p-(p-iodophénoxy)phénoxy]-propionique,

/[(3,3,5-triméthylcyclohexylidèneamino)oxy]méthyl/-ester de l'acide D-2-/p-[(α,α,α-trifluoro-p-tolyl)oxy]phénoxy/-propionique et

/[(1,3-diméthyl-2-buténylidèneamino)oxy]-méthyl/ester de l'acide D-2-/p-[(α,α,α-trifluoro-p-tolyl)-oxy]phénoxy/-propionique, et un support inerte.

12. Agent de lutte contre les mauvaises herbes selon la revendication 1 où l'on utilise comme composé(s) de formule I les isomères D.

13. Agent de lutte contre les mauvaises herbes selon l'une des revendications 2 à 7 où l'on utilise comme composé(s) de formule I les isomères D.

14. Procédé de préparation de composés de formule générale

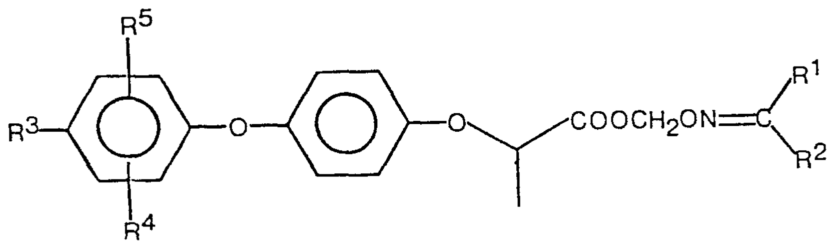

(I)

où $R^1$ représente un hydrogène, un alcoyle en $C_1$ à $C_6$ ou un phényle, $R^2$ représente un alcoyle en $C_1$ à $C_6$, un alcényle en $C_2$ à $C_6$ ou un phényle, ou bien où $R^1$ et $R^2$ forment ensemble avec l'atome de carbone auquel ils sont liés un noyau cycloalcane en $C_4$ à $C_{10}$, qui peut éventuellement être mono-, di- ou trisubstitué par un alcoyle en $C_1$ à $C_3$, $R^3$ représente un halogène, un trifluorométhyle ou un nitro et $R^4$ et $R^5$ représente un hydrogène ou un halogène, caractérisé en ce que

a) on fait réagir un sel d'un acide de formule

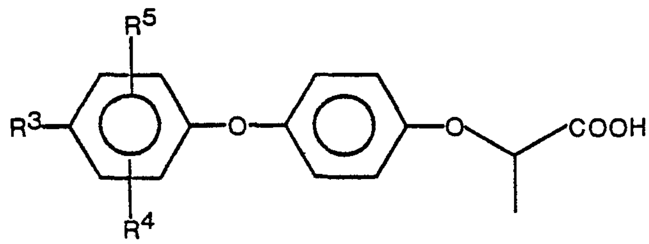

(II)

où $R^3$, $R^4$ et $R^5$ ont la signification donnée pour la formule I, avec un composé de formule

$$R^1R^2CNOCH_2X \qquad \text{(III)}$$

où $R^1$ et $R^2$ ont la signification donnée pour la formule I et X représente un groupe sortant, ou

b) on fait réagir un composé de formule

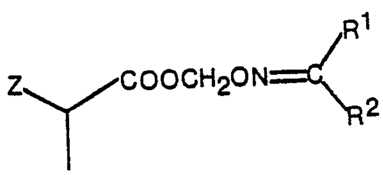

(IV)

où Z représente un groupe sortant et $R^1$ et $R^2$ ont la signification donnée pour la formule I, avec un composé de formule

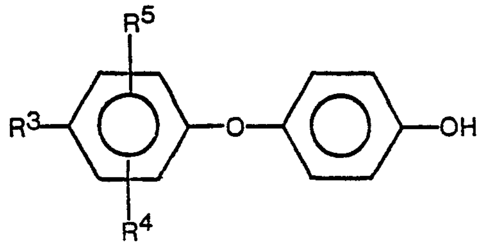

(V)

où $R^3$, $R^4$ et $R^5$ ont la signification donnée pour la formule I, ou un de ses sels de métaux alcalins, ou

c) on fait réagir un composé de formule

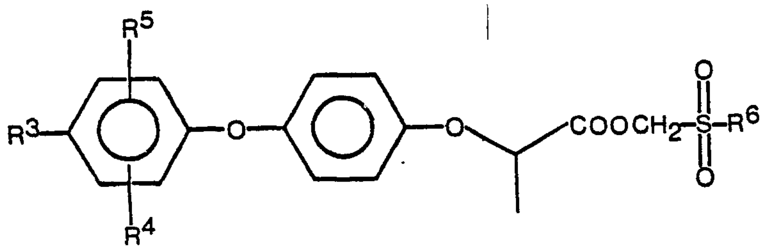 (VI)

où $R^6$ représente un groupe alcoyle inférieur, aryle ou hétéroaryle et $R^3$, $R^4$ et $R^5$ ont la signification donnée pour la formule I, avec un composé de formule

$$R^1R^2CNOH \qquad (VII)$$

où $R^1$ et $R^2$ ont la signification donnée pour la formule I, ou un de ses sels de métaux alcalins en présence d'une base.

15. Procédé selon la revendication 14 de préparation de composés de formule générale I selon la revendication 14, où $R^1$ représente un hydrogène, un alcoyle en $C_1$ à $C_6$ ou un phényle, $R^2$ représente un alcoyle en $C_1$ à $C_6$, un alcényle en $C_2$ à $C_6$, un alcynyle en $C_2$ à $C_6$ ou un phényle, ou bien où $R^1$ et $R^2$ forment ensemble avec l'atome de carbone auquel ils sont liés un noyau cycloalcane en $C_4$ à $C_6$, qui peut être éventuellement mono-, di- ou trisubstitué par un alcoyle en $C_1$ à $C_3$, $R^3$ représente un halogène, un trifluorométhyle ou un nitro et $R^4$ et $R^5$ représentent un hydrogène ou un halogène, et X et Z représentent un chlore, un brome, un iode, un mésyloxy ou un tosyloxy.

16. Procédé selon la revendication 15 de préparation de l'/[(isopropylidèneamino)oxy]méthyl/ester de l'acide 2-/p-[(α,α,α-trifluoro-p-tolyl)oxy]phénoxy/propionique, caractérisé en ce qu'on fait réagir le [(méthylsulfonyl)méthyl]ester de l'acide 2-/p-[(α,α,α-trifluoro-p-tolyl)oxy]-phénoxy/-propionique avec le sel de sodium de l'acétonoxime.

17. Procédé selon la revendication 14 de préparation de l'/[(isopropylidèneamino)oxy]méthyl/ester de l'acide 2-[p-(p-chlorophénoxy)phénoxy]-propionique, caractérisé en ce qu'on fait réagir le [(méthylsulfonyl)méthyl]ester de l'acide 2-[p-(p-chlorophénoxy)phénoxy]-propionique avec le sel de sodium de l'acétonoxime.

18. Procédé selon la revendication 14 ou 17, caractérisé en ce qu'on utilise le produit de départ de formule VI sous forme D.

19. Procédé selon la revendication 15 ou 16, caractérisé en ce qu'on utilise le produit de départ de formule VI sous forme D.

20. Application d'un composé ou agent mentionné dans la revendication 1, 11 ou 12 à la lutte contre les mauvaises herbes.

21. Application d'un des composés ou agents mentionnés dans les revendications 2 à 10 et 13 à la lutte contre les mauvaises herbes.